# EUROPEAN PATENT APPLICATION

(11) **EP 3 486 656 A1**
(43) Date of publication of application: **22.05.2019**
(21) Application number: 18193539.6
(22) Date of filing: 22.06.2012
(51) Int. Cl.: G01N 33/558, G01N 33/573, C12Q 1/42

(54) **ASSAY**

(30) Priority: 07.07.2011 GB 201111634; 07.10.2011 GB 201117309; 10.10.2011 US 201161545237 P; 12.10.2011 EP 11184930
(62) Divisional of application: 12731634.7
(71) Applicant: DuPont Nutrition Biosciences ApS, 1411 Køpenhaven K (DK)
(72) Inventor: ISAKSEN, Mai Faurschou, 8270 Højbjerg (DK); KELLETT-SMITH, Anja Hemmingsen, Copenhagen V 1558 (DK); MCLENNAN, Neil, Fife, Scotland, KY16 0DZ (GB); GILBERT, Ceinwen, Corsham, Wiltshire SN13 0LY (GB); KYNEB, Majbritt Hauge, DK-8260 Viby J (DK)
(74) Representative: D Young & Co LLP

(57) **Abstract**

The present invention discloses an assay device (1) for detecting active enzyme in a sample. Said assay device (1) comprises the following components:- (a) a placement region (10) onto which the sample can be placed; (b) a matrix (20) operably connected to said placement region (10) such that the sample when present (such as placed) on said placement region (10) can migrate along said matrix (20); (c) at least one distinct capture location (30) on said matrix (20), wherein each distinct capture location (30) is distanced away from the placement region (10), and wherein the sample can migrate across said distinct capture location (30); (d) capture means (40) being present at or defining each distinct capture location (30), wherein said capture means (40) are capable of binding to said enzyme such that at least a portion of said sample of said enzyme is retained at at least one distinct capture location (30); and (e) selective indication means (50), or at least a component thereof, to provide selective indication of the presence of active enzyme bound to said capture means (40).

SEQUENCE LISTING

<110>  Danisco A/S

<120>  ASSAY

<130>  P043539EP1A

<150>  11184930.3
<151>  2011-10-12

<150>  GB 1111634.0
<151>  2011-07-07

<150>  GB 1117309.3
<151>  2011-10-07

<150>  US 61/545237
<151>  2011-10-10

<160>  4

<170>  PatentIn version 3.5

<210>  1
<211>  413
<212>  PRT
<213>  Buttiauxella sp.

<400>  1

Asn Asp Thr Pro Ala Ser Gly Tyr Gln Val Glu Lys Val Val Ile Leu
1               5                   10                  15

Ser Arg His Gly Val Arg Ala Pro Thr Lys Met Thr Gln Thr Met Arg
            20                  25                  30

Asp Val Thr Pro Asn Thr Trp Pro Glu Trp Pro Val Lys Leu Gly Tyr
        35                  40                  45

Ile Thr Pro Arg Gly Glu His Leu Ile Ser Leu Met Gly Gly Phe Tyr
    50                  55                  60

Arg Gln Lys Phe Gln Gln Gln Gly Ile Leu Ser Gln Gly Ser Cys Pro
65                  70                  75                  80

Thr Pro Asn Ser Ile Tyr Val Trp Thr Asp Val Ala Gln Arg Thr Leu
                85                  90                  95

Lys Thr Gly Glu Ala Phe Leu Ala Gly Leu Ala Pro Gln Cys Gly Leu
            100                 105                 110

Thr Ile His His Gln Gln Asn Leu Glu Lys Ala Asp Pro Leu Phe His
        115                 120                 125

Pro Val Lys Ala Gly Ile Cys Ser Met Asp Lys Thr Gln Val Gln Gln
    130                 135                 140

Ala Val Glu Lys Glu Ala Gln Thr Pro Ile Asp Asn Leu Asn Gln His
145                 150                 155                 160

Tyr Ile Pro Ser Leu Ala Leu Met Asn Thr Thr Leu Asn Phe Ser Lys
                165                 170                 175

Ser Pro Trp Cys Gln Lys His Ser Ala Asp Lys Ser Cys Asp Leu Gly
            180                 185                 190

Leu Ser Met Pro Ser Lys Leu Ser Ile Lys Asp Asn Gly Asn Glu Val
        195                 200                 205

Ser Leu Asp Gly Ala Ile Gly Leu Ser Ser Thr Leu Ala Glu Ile Phe
    210                 215                 220

Leu Leu Glu Tyr Ala Gln Gly Met Pro Gln Ala Ala Trp Gly Asn Ile
225                 230                 235                 240

His Ser Glu Gln Glu Trp Ala Leu Leu Leu Lys Leu His Asn Val Tyr
                245                 250                 255

Phe Asp Leu Met Glu Arg Thr Pro Tyr Ile Ala Arg His Lys Gly Thr
            260                 265                 270

Pro Leu Leu Gln Ala Ile Ser Asn Ala Leu Asn Pro Asn Ala Thr Glu
        275                 280                 285

Ser Lys Leu Pro Asp Ile Ser Pro Asp Asn Lys Ile Leu Phe Ile Ala
    290                 295                 300

Gly His Asp Thr Asn Ile Ala Asn Ile Ala Gly Met Leu Asn Met Arg
305                 310                 315                 320

Trp Thr Leu Pro Gly Gln Pro Asp Asn Thr Pro Pro Gly Gly Ala Leu
                325                 330                 335

Val Phe Glu Arg Leu Ala Asp Lys Ser Gly Lys Gln Tyr Val Ser Val
            340                 345                 350

Ser Met Val Tyr Gln Thr Leu Glu Gln Leu Arg Ser Gln Thr Pro Leu
        355                 360                 365

Ser Leu Asn Gln Pro Ala Gly Ser Val Gln Leu Lys Ile Pro Gly Cys
    370                 375                 380

Asn Asp Gln Thr Ala Glu Gly Tyr Cys Pro Leu Ser Thr Phe Thr Arg
385                 390                 395                 400

Val Val Ser Gln Ser Val Glu Pro Gly Cys Gln Leu Gln
                405                 410

<210>  2
<211>  413
<212>  PRT
<213>  Buttiauxella sp.

<400>  2

Asn Asp Thr Pro Ala Ser Gly Tyr Gln Val Glu Lys Val Val Ile Leu
1               5                   10                  15

Ser Arg His Gly Val Arg Ala Pro Thr Lys Met Thr Gln Thr Met Arg
            20                  25                  30

Asp Val Thr Pro Asn Thr Trp Pro Glu Trp Pro Val Lys Leu Gly Tyr
        35                  40                  45

Ile Thr Pro Arg Gly Glu His Leu Ile Ser Leu Met Gly Gly Phe Tyr
    50                  55                  60

Arg Gln Lys Phe Gln Gln Gln Gly Ile Leu Ser Gln Gly Ser Cys Pro
65                  70                  75                  80

Thr Pro Asn Ser Ile Tyr Val Trp Thr Asp Val Asp Gln Arg Thr Leu
                85                  90                  95

Lys Thr Gly Glu Ala Phe Leu Ala Gly Leu Ala Pro Gln Cys Gly Leu
            100                 105                 110

Thr Ile His His Gln Gln Asn Leu Glu Lys Ala Asp Pro Leu Phe His
        115                 120                 125

Pro Val Lys Ala Gly Ile Cys Ser Met Asp Lys Thr Gln Val Gln Gln
    130                 135                 140

Ala Val Glu Lys Glu Ala Gln Thr Pro Ile Asp Asn Leu Asn Gln His
145                 150                 155                 160

Tyr Ile Pro Ser Leu Ala Leu Met Asn Thr Thr Leu Asn Phe Ser Lys
                165                 170                 175

Ser Pro Trp Cys Gln Lys His Ser Ala Asp Lys Ser Cys Asp Leu Gly
            180                 185                 190

Leu Ser Met Pro Ser Lys Leu Ser Ile Lys Asp Asn Gly Asn Glu Val
        195                 200                 205

Ser Leu Asp Gly Ala Ile Gly Leu Ser Ser Thr Leu Ala Glu Ile Phe
    210                 215                 220

Leu Leu Glu Tyr Ala Gln Gly Met Pro Gln Ala Ala Trp Gly Asn Ile
225                 230                 235                 240

His Ser Glu Gln Glu Trp Ala Leu Leu Leu Lys Leu His Asn Val Tyr
                245                 250                 255

Phe Asp Leu Met Glu Arg Thr Pro Tyr Ile Ala Arg His Lys Gly Thr
            260                 265                 270

Pro Leu Leu Gln Ala Ile Ser Asn Ala Leu Asn Pro Asn Ala Thr Glu
        275                 280                 285

Ser Lys Leu Pro Asp Ile Ser Pro Asp Asn Lys Ile Leu Phe Ile Ala
    290                 295                 300

Gly His Asp Thr Asn Ile Ala Asn Ile Ala Gly Met Leu Asn Met Arg
305                 310                 315                 320

Trp Thr Leu Pro Gly Gln Pro Asp Asn Thr Pro Pro Gly Gly Ala Leu
                325                 330                 335

Val Phe Glu Arg Leu Ala Asp Lys Ser Gly Lys Gln Tyr Val Ser Val
            340                 345                 350

Ser Met Val Tyr Gln Thr Leu Glu Gln Leu Arg Ser Gln Thr Pro Leu
        355                 360                 365

Ser Leu Asn Gln Pro Ala Gly Ser Val Gln Leu Lys Ile Pro Gly Cys
    370                 375                 380

Asn Asp Gln Thr Ala Glu Gly Tyr Cys Pro Leu Ser Thr Phe Thr Arg
385                 390                 395                 400

Val Val Ser Gln Ser Val Glu Pro Gly Cys Gln Leu Gln
                405                 410

<210>  3
<211>  413
<212>  PRT
<213>  Buttiauxella sp.

<400>  3

Asn Asp Thr Pro Ala Ser Gly Tyr Gln Val Glu Lys Val Val Ile Leu
1               5                   10                  15

Ser Arg His Gly Val Arg Ala Pro Thr Lys Met Thr Gln Thr Met Arg
            20                  25                  30

Asp Val Thr Pro Tyr Thr Trp Pro Glu Trp Pro Val Lys Leu Gly Tyr
        35                  40                  45

Ile Thr Pro Arg Gly Glu His Leu Ile Ser Leu Met Gly Gly Phe Tyr
    50                  55                  60

Arg Gln Lys Phe Gln Gln Gln Gly Ile Leu Pro Arg Gly Ser Cys Pro
65                  70                  75                  80

Thr Pro Asn Ser Ile Tyr Val Trp Thr Asp Val Ala Gln Arg Thr Leu
                85                  90                  95

Lys Thr Gly Glu Ala Phe Leu Ala Gly Leu Ala Pro Gln Cys Gly Leu
            100                 105                 110

Thr Ile His His Gln Gln Asn Leu Glu Lys Ala Asp Pro Leu Phe His
        115                 120                 125

Pro Val Lys Ala Gly Ile Cys Ser Met Asp Lys Thr Gln Val Gln Gln
    130                 135                 140

Ala Val Glu Lys Glu Ala Gln Thr Pro Ile Asp Asn Leu Asn Gln Arg
145                 150                 155                 160

Tyr Ile Pro Glu Leu Ala Leu Met Asn Thr Ile Leu Asn Phe Ser Lys
                165                 170                 175

Ser Pro Trp Cys Gln Lys His Ser Ala Asp Lys Pro Cys Asp Leu Ala
            180                 185                 190

Leu Ser Met Pro Ser Lys Leu Ser Ile Lys Asp Asn Gly Asn Glu Val
        195                 200                 205

Ser Leu Asp Gly Ala Ile Gly Leu Ser Ser Thr Leu Ala Glu Ile Phe
    210                 215                 220

Leu Leu Glu Tyr Ala Gln Gly Met Pro Gln Val Ala Trp Gly Asn Ile
225                 230                 235                 240

His Ser Glu Gln Glu Trp Ala Leu Leu Leu Lys Leu His Asn Val Tyr
                245                 250                 255

Phe Asp Leu Met Glu Arg Thr Pro Tyr Ile Ala Arg His Lys Gly Thr
            260                 265                 270

Pro Leu Leu Gln Ala Ile Ser Asn Ala Leu Asn Pro Asn Ala Thr Glu
        275                 280                 285

Ser Lys Leu Pro Asp Ile Ser Pro Asp Asn Lys Ile Leu Phe Ile Ala
    290                 295                 300

Gly His Asp Thr Asn Ile Ala Asn Ile Ala Gly Met Leu Asn Met Arg
305                 310                 315                 320

Trp Thr Leu Pro Gly Gln Pro Asp Asn Thr Pro Pro Gly Gly Ala Leu
                325                 330                 335

Val Phe Glu Arg Leu Ala Asp Lys Ser Gly Lys Gln Tyr Val Ser Val
            340                 345                 350

Ser Met Val Tyr Gln Thr Leu Glu Gln Leu Arg Ser Gln Thr Pro Leu
        355                 360                 365

Ser Leu Asn Gln Pro Pro Gly Ser Val Gln Leu Lys Ile Pro Gly Cys
    370                 375                 380

Asn Asp Gln Thr Ala Glu Gly Tyr Cys Pro Leu Ser Thr Phe Thr Arg
385                 390                 395                 400

Val Val Ser Gln Ser Val Glu Pro Gly Cys Gln Leu Gln
                405                 410

<210>  4
<211>  7
<212>  PRT
<213>  Artificial Sequence

<220>
<223>  HAP phytase active site motif


<220>
<221>  misc_feature
<222>  (4)..(4)
<223>  Xaa can be any naturally occurring amino acid

<220>
<221>  misc_feature
<222>  (6)..(6)
<223>  Xaa can be any naturally occurring amino acid

<400>  4

Arg His Gly Xaa Arg Xaa Pro
1               5

## Description

### FIELD

The present invention relates to the field of enzyme assays.

In particular, the present invention relates to enzyme activity strip assays.

More specifically, the present invention relates to assay devices and methods using same and uses of same. The assay devices and assays can detect active enzyme. The active enzyme may be a phosphatase, such as a phytase or an acid phosphatase, or a glycoside hydrolase such as a xylanase, a β-glucanase, or an amylase. In a more preferred aspect, the active enzyme is a phytase.

The present invention is useful for application in a variety of industrial applications, such as detecting active enzyme in biofuel production, detergent compositions and detecting active enzyme in foods and animal feeds.

### BACKGROUND

Determining the presence or absence of an analyte, particularly an enzyme, is frequently necessary in the laboratory, and also in fields as diverse as food and animal feeds, biofuel production and washing soaps and powders. Commonly, assays such as immunoassays are used for this purpose.

Enzyme-linked immunosorbent assays (ELISAs) can be used to detect the presence of an antibody or antigen in a sample. In such methods, an antibody binds to an antigen which is fixed to a surface, or vice versa. This antibody is linked to an enzyme which can create detectable signal, which is usually a colour change.

Another common type of assay or immunoassay is the lateral flow assay. In such assays the presence of an analyte is detected in a sample which flows along a solid support. The analyte, or a reagent such as an antibody which is bound to the analyte, then binds to lines or zones in the support which have been treated with an antigen or antibodies.

Both ELISAs and lateral flow assays can also be sandwich assays. This means the sample first contacts a capture antibody which binds to the antigen to be detected. The antigen then binds to a further antibody, which is likely to be at a fixed test line in the case of lateral flow assays. The antigen is thus sandwiched between at least two antibodies. The second antibody is linked to a detectable signal.

Many assays or immunoassays of the types described above determine the presence of a specific enzyme.

However, in some samples the enzyme exists but has been inactivated, for example by heat or chemical inactivation. In such cases, existing assays still detect the presence of the enzyme.

For example, US4425438 describes an assay which uses a column rather than lateral flow system. The sample flows through zones of beads which are coated with an analyte absorbent. The zones in which analyte binding occurs gives a measure of analyte quantity. Another column assay is described in US5073484, wherein the quantitative determination of an analyte in liquid takes place using spaced zones along the flow path.

Lateral flow devices, such as e.g. that described in US5451504, commonly have different zones for deposit of the sample, trapping or immobilisation of the analyte and detection of the trapped analyte or analyte complex. Such devices do not quantify the analyte or identify activity if the analyte is an enzyme.

The lateral flow assay disclosed in US6183972 uses multiple capture locations or bands to capture labelled anti-analyte antibody and provide a detectable signal. The bands provide a unique pattern of signals, which, when mathematically combined in order to create a monotonous dose-response curve, indicates the concentration of the analyte in the sample.

US5229073 describes a lateral flow assay which uses multiple capture locations in order to semi-quantify the amount of analyte in a sample.

Large molecules (e.g. >3000 daltons) can be detected using the lateral flow device of US6358548. Depending on the embodiment, the colour intensity of number of lines at capture sites gives an indication of the quantity of the analyte.

US7425302 discloses a lateral flow method wherein the reactants are dried. Adding the liquid sample re-constitutes the dry reactants. This method quantifies the analyte based on the intensity of a colour change. The activity of the specific enzyme G6PD is determined by the length of time it takes for a colour change to occur, which is a measure of how quickly G6PD reacts to its substrate. However, the method of US7425302 is limited specifically to G6PD and no enzyme is retained at a distinct capture location.

WO2005/014847 describes the use of a standard lateral flow sandwich immunoassay to detect enzymes such as phytases, xylanases and amylases. The quantity of the enzymes can be at least partially determined by the intensity of the resulting colour change. WO2007/001895 describes a colloidal gold sandwich assay which is used specifically to detect *E. coli* phytase. This colloidal gold sandwich assay comprises a primary monoclonal antibody which immunologically recognises the phytase and a secondary antibody which is conjugated to a means of detection.

Accordingly, some of the assays used previously may only provide quantitative determination of an enzyme in a sample, but are not able to determine the activity of the enzyme or do not specifically identify an active enzyme. Other assays require complicated mathematical analysis of the capture bands in order to determine an analyte concentration.

There is therefore a need for an assay which detects only active forms of an enzyme. It would also be advantageous if such an assay could provide at least some semi-quantitative information.

The present invention seeks to overcome some of the problems of the prior art devices and methods.

The present invention will now be described. For ease of reference we have described elements of the present invention under one or more headings. It is to be noted that the teachings under each of the headings also applies to the teachings under the other headings. For example, each of the stated preferred elements and aspects concerning the device of the present invention is equally a preferred element or aspect concerning the method of the present invention or the use of the present invention. Likewise, each of the stated preferred elements and aspects concerning the method or use of the present invention is equally a preferred element or aspect concerning the device of the present invention.

### SUMMARY ASPECTS

In a broad aspect, the present invention relates to an enzyme assay and a device which detects active enzyme in a sample, and to methods for determining the presence of active enzyme in a sample.

In particular, the present invention relates to devices and kits for performing such assays, specifically lateral flow assays and devices for performing lateral flow assays and immunoassays.

### GENERAL EMBODIMENTS

In one embodiment, the present invention provides an assay device for detecting active enzyme in a sample.

The device comprises: (a) a placement region onto which the sample can be placed; (b) a matrix operably connected to said placement region such that the sample when present (such as placed) on said placement region can migrate along said matrix; (c) at least one distinct capture location on said matrix, wherein each distinct capture location is distanced away from the placement region, and wherein the sample can migrate across said distinct capture location; (d) capture means being present at or defining each distinct capture location, wherein said capture means are capable of binding to said enzyme such that at least a portion of said sample of said enzyme is retained at at least one distinct capture location; and (e) selective indication means or at least a component thereof to provide selective indication of the presence of active enzyme bound to said capture means.

In another embodiment, the present invention provides a method of determining the presence of active enzyme in a sample wherein the sample is placed on the placement region of the assay device of the invention and the selective indication means of the device indicate if active enzyme is present.

In another embodiment, the present invention provides a method of determining active enzyme levels in a sample wherein the sample is placed on the placement region of the assay device of the invention and the selective indication means of the device indicate the amount of active enzyme present semi-quantitatively.

In another embodiment, the present invention provides a kit comprising or capable of forming the assay device of the present invention.

In another embodiment the present invention provides a method of determining the presence of active enzyme in a sample wherein the method comprises the steps of:
a) contacting at least a portion of the sample with a capture means whereby the enzyme is captured by said capture means;
b) providing a suitable substrate for the captured enzyme wherein said substrate reacts with said captured enzyme to yield an enzyme product;
c) providing a reactive species capable of reacting with the enzyme product wherein said reactive species reacts with said enzyme product to yield an insoluble product; and
d) detecting the insoluble product obtained in step c) wherein said detecting correlates to the presence and/or quantity of an active enzyme in said sample.

### SOME ADVANTAGES

An advantage of the present invention is that the device and methods using same enable an operator to identify active enzyme in a sample.

An additional advantage of the present invention is that the device and methods using same enable an operator in a simple manner to identify active enzyme in a sample and, in some instances, at least in a semi-quantitative manner.

The term "semi-quantitative" as used herein means a relative quantity, an approximate quantity or a quantity within a known range.

Preferably the term "semi-quantitative" means an estimate of the level of active enzyme, i.e. "low", "acceptable" or "high" level of active enzyme. It does however not exclude a precise quantitative determination of units. This could, for example, be obtained by more distinct capture locations in the capture regions or by measuring or observing the intensity of a colour formation or by measuring or observing a colour change.

Additional advantages of the present invention are mentioned herein.

### GENERAL ASPECTS

In one aspect, the assay device comprises a placement region where a sample can be placed. Preferably the sample is a liquid sample. Preferably the same is a food or feed sample or a sample comprising a food or feed component. A matrix is operably connected to the placement region such that the sample can migrate from the placement region along the matrix. Preferably the matrix is a material such as nitrocellulose.

The matrix of the assay device of the current application comprises at least one distinct capture location which is distanced away from the placement region. The sample can migrate across the capture location. Preferably at least two capture locations are used. Preferably at least three capture locations are used. Preferably three capture locations are used.

At each capture location in the assay device of the current invention, capture means are present. The capture means are capable of binding to the enzyme to be detected by the assay device. At least a sample of the enzyme is retained at at least one capture location. The capture means are preferably antibodies and most preferably the capture means are antibodies that bind to the enzyme. The antibodies may be raised against the enzyme *per se* or against a component thereof (e.g. a fragment thereof and/or an epitope thereof).

Selective indication means are used in the assay device of the current invention to provide selective indication of the presence of active enzyme bound to the capture means. Preferably the selective indication means comprises a precipitation reaction and/or provides a measurable change in visible or UV wavelength which occurs upon product formation. The colour change may be caused by the reaction of a substrate with the enzyme. Reaction with a substrate indicates the enzyme is active and not merely present in an inactive or denatured form.

Preferably, a visible colour change means a colour change that can be observed by the naked eye or by a mechanical reader and/or by an electronic reader.

All or at least some components of the selective indication means are present in the assay device. For some embodiments all of the selective indication means are present in and/or on the assay device. In other embodiments, the assay device comprises at least one of the components of the selective indication means and the device is supplied with (e.g. as a kit) with the other component(s) of the selective indication means. Thus, in use, the user has all of the requisite components of the selective indication means to use the device to assay for active enzyme.

The present invention is advantageous as it provides a novel and a simple assay which can be used to detect active enzyme. This is particularly useful in the bioethanol, detergent and food and feed industries where enzymes may be detected as present using currently available assays, but be inaccessible - for example due to inactivation due to heat or chemical treatment. Furthermore, the assays and methods of the invention may be useful in determining whether additional active enzyme of a feed or food additive is needed. Such a method could also be used for quality control of a product such as a food or feed product.

Another aspect of the invention is it provides a method to determine semi-quantitatively the amount of active enzyme in a sample. Preferably this semi-quantitative approach is relative to the number of capture regions which indicate the presence of bound enzyme.

A further aspect of this invention is that it provides a method of determining levels of active enzyme in a food or feed, and a method of determining whether additional enzyme or a feed or food additive is needed. Such a method could also be used for quality control of a product such as a food or feed product.

The current invention may be presented in the form of a kit. Such a kit will enable the operator to perform the assay, preferably outside of the laboratory. The kit may provide all the reagents necessary to perform the assay. All or some of such reagents may be in a dried form.

Preferably the active enzyme detected by the assay or assay device of the current invention is a phosphatase or a glycoside hydrolase, preferably an acid phosphatase, a phytase, a xylanase, an amylase, or a β-glucanase.

In a more preferred aspect, the active enzyme detected by the assay or assay device of the current invention is a phosphatase, preferably an acid phosphatase, more preferably a phytase.

The term "phytase" means a protein or polypeptide which is capable of catalysing the hydrolysis of esters of phosphoric acid, including phytate/phytic acid, and releasing inorganic phosphate. Some phytases in addition to phytate are capable of hydrolysing at least some of the inositol-phosphates of intermediate degrees of phosphorylation.

The non-starch polysaccharide (NSP) fraction of some cereals such as wheat and barley increases viscosity in the gut, which compromises diffusion of nutrients. This anti-nutritional effect can be reduced by addition of the xylanase and/or beta-glucanase, which fragment hemi-cellulose polymers, xylan and beta-glucan, respectively.

Alpha-amylases amongst other supplements are added to feed to improve the starch utilisation of the feed.

Phytase enzymes, such as e.g. the 6-phytase BP17 derived from *Buttiauxella sp.,* are added to animal feed to increase phosphate availability thus increasing the nutritional value of the product. The processing of the feed, for example under heat and high pressure, can denature the phytase and reduce its activity. The present invention provides a semi-quantitative test that can specifically give an indication of the post-processing levels of phytase activity present in the feed.

In a highly preferred aspect, the active enzyme detected by the current invention is a phytase, more in particular a 6-phytase.

In a highly preferred aspect, the active enzyme to be detected is BP17. BP17 is an enzyme variant of a *Buttiauxella sp.* phytase. The sequence for BP17 (excluding signal peptide) is as follows:

NDTPASGYQVEKVVILSRHGVRAPTKMTQTMRDVTPNTWPEWPVKLGYITPRGEHLISLMGGFYRQKFQQQGILSQGSCPTPNSI
YVWTDVAQRTLKTGEAFLAGLAPQCGLTIHHQQNLEKADPLFHPVKAGICSMDKTQVQQAVEKEAQTPIDNLNQHYIPSLALMNT
TLNFSKSPWCQKHSADKSCDLGLSMPSKLSIKDNGNEVSLDGAIGLSSTLAEIFLLEYAQGMPQAAWGNIHSEQEWALLLKLHNV
YFDLMERTPYIARHKGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPAGSVQLKIPGCNDQTAEGYCPLSTFTRVVSQSVEPGCQLQ

In another embodiment, the active enzyme to be detected is BP11. BP11 is an enzyme variant of a *Buttiauxella sp.* phytase. BP11 is used in bioethanol production. The sequence for BP11 (excluding signal peptide) is as follows:

NDTPASGYQVEKVVILSRHGVRAPTKMTQTMRDVTPNTWPEWPVKLGYITPRGEHLISLMGGFYRQKFQQQGILSQGSCPTPNSI
YVWTDVDQRTLKTGEAFLAGLAPQCGLTIHHQQNLEKADPLFHPVKAGICSMDKTQVQQAVEKEAQTPIDNLNQHYIPSLALMNT
TLNFSKSPWCQKHSADKSCDLGLSMPSKLSIKDNGNEVSLDGAIGLSSTLAEIFLLEYAQGMPQAAWGNIHSEQEWALLLKLHNV
YFDLMERTPYIARHKGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPAGSVQLKIPGCNDQTAEGYCPLSTFTRVVSQSVEPGCQLQ

In another embodiment, the active enzyme to be detected is BP111. BP111 is an enzyme variant of a *Buttiauxella sp.* phytase. BP111 is used in bioethanol production. The sequence for BP111 (excluding signal peptide) is as follows:

NDTPASGYQVEKVVILSRHGVRAPTKMTQTMRDVTPYTWPEWPVKLGYITPRGEHLISLMGGFYRQKFQQQGILPRGSCPTPNSI
YVWTDVAQRTLKTGEAFLAGLAPQCGLTIHHQQNLEKADPLFHPVKAGICSMDKTQVQQAVEKEAQTPIDNLNQRYIPELALMNT
ILNFSKSPWCQKHSADKPCDLALSMPSKLSIKDNGNEVSLDGAIGLSSTLAEIFLLEYAQGMPQVAWGNIHSEQEWALLLKLHNV
YFDLMERTPYIARHKGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPPGSVQLKIPGCNDQTAEGYCPLSTFTRVVSQSVEPGCQLQ

### BRIEF DESCRIPTION OF THE FIGURES

The present invention will be described by reference to the following Figures:
**Figure 1** - presents two diagrams.
**Figure 2** - presents a diagram (Format 1: Enzyme Capture and Precipitating Substrate Assay).
**Figure 3** - presents a series of photographs (Steps involved in the Semi-Dry Format).
**Figure 4** - presents a photograph (Results from a phytase BP17 dilution series using the Semi-Dry Format).
**Figure 5** - presents a diagram (Format 2: Gold Conjugate Antibody Sandwich Assay)**.**
**Figure 6** - presents a series of photographs (An example of a Dry Format test).
**Figure 7** - presents a series of photographs (Results for samples prepared in acetate buffer, MilliQ water and tap water).
**Figure 8** - presents a series of photographs (Sampling at different time intervals).
**Figure 9** - presents a series of photographs (SDS-PAGE and Western blotting of feed samples and purified phytase BP17).
**Figure 10** - presents a series of photographs (Comparison of the Gold Sandwich format and the Precipitating Substrate enzyme activity format using phytase BP17 specific inhibitor MIHS).
**Figure 11** - presents a diagram and a photograph (Schematic representation of the Flow Through Format).
**Figure 12** - presents a diagram (Schematic representation of the Dip Stick Format).
**Figure 13** - presents a photograph (Dip-stick Format test results)
**Figure 14** - presents a diagram (Schematic representation of the Wicking Stick Format).
**Figure 15** - presents a series of photographs (Wicking Stick Format test results).
**Figure 16** - presents a series of photographs (Wicking Stick Format test results using different blocking agents).
**Figure 17** - presents a series of photographs (Varying the test line concentration to modulate the semi-quantitative nature of the test. Wicking Stick Format).
**Figure 18** - presents a series of photographs (Comparison between NBT and INT using the Wicking Stick Format).
**Figure 19** - presents a series of photographs (Testing a wide range of nitrocellulose types in order to determine the most appropriate).
**Figure 20** - presents a photograph (Testing reducing the concentration of blocking agent casein in the presence of surfactant (Tween-20)).
**Figure 21** - presents a series of photographs (Adding the blocking reagents directly onto the test nitrocellulose to test if satisfactory results could be obtained).
**Figure 22** - presents a series of photographs (A dilution series of phytase BP17 concentrations tested with surfactant-only blocked NC with both INT and NBT substrates to test the sensitivity of this method).
**Figure 23** - presents a series of photographs (Testing drying and storing the various substrate components mixed together).
**Figure 24** - presents a series of photographs (A comparison between the Semi-Dry Format and the All Solution format).
**Figure 25** - presents a series of photographs (Periodically testing solutions for activity suggests that the tetrazolium solution has good stability).
**Figure 26** - presents a photograph of testing solutions for xylanase activity.
**Figure 27** - presents a photograph of testing solutions for xylanase activity.
**Figure 28** - presents a schematic diagram for testing for active xylanase activity.
**Figure 29** - presents a series of schematic diagrams.

### DETAILED DESCRIPTION

The present invention provides a device for detecting active enzyme. The device can be used in the laboratory and also outside of the laboratory in diverse fields.

The present invention provides an assay device (1) for detecting active enzyme in a sample. The assay device (1) comprises the following components: (a) a placement region (10) onto which the sample can be placed; (b) a matrix (20) operably connected to said placement region (10) such that the sample when present (such as placed) on said placement region (10) can migrate along said matrix (20); (c) at least one distinct capture location (30) on said matrix (20), wherein each distinct capture location (30) is distanced away from the placement region (10), and wherein the sample can migrate across said distinct capture location (30); (d) capture means (40) being present at or defining each distinct capture location (30), wherein said capture means (40) are capable of binding to said enzyme such that at least a portion of said sample of said enzyme is retained at at least one distinct capture location (30); and (e) selective indication means (50) or at least a component thereof to provide selective indication of the presence of active enzyme bound to said capture means (40).

Detecting the presence of an active enzyme, and the amount of such an enzyme, is of particular importance in the field of food and feed. The absence of active enzyme may potentially lead to nutrient and mineral deficiencies in humans and animals which ingest the food or feed. The ability to detect the presence of an enzyme may not equate to the ability to detect the presence of active enzyme using existing methods.

As a particular example, phytate is the major storage form of phosphorus in cereals and legumes. However, monogastric animals such as pig, poultry and fish are not able to metabolise or absorb phytate (or phytic acid) and therefore it is excreted, leading to phosphorous pollution in areas of intense livestock production. Moreover, phytic acid also acts as an anti-nutritional agent in monogastric animals by chelating metal agents such as calcium, copper and zinc.

Through the action of phytase, phytate is generally hydrolysed to give lower inositol-phosphates and inorganic phosphate. Phytases are useful as additives to animal feeds where they improve the availability of organic phosphorus to the animal and decrease phosphate pollution of the environment (Wodzinski RJ, Ullah AH. Adv Appl Microbiol. 42, 263-302 (1996)). However, if the phytase contained in a feed is inactive, this will have no phytase hydrolysing effect, which could lead to malnutrition in animals. The current invention therefore enables an operator such as an animal keeper, farmer, veterinarian, zoo keeper, feed additive producer, feed producer, scientist or member of the public to test an animal feed to see if it contains active enzyme, such as e.g. phytase, and the amount of active enzyme, such as phytase.

The current invention could also be used in a method of quality control in the preparation of feeds or food. biofuel production or detergent compositions to which an enzyme, such as a phosphatase or a glycoside hydrolase, preferably an acid phosphatase, a phytase, a xylanase, an amylase, or a β-glucanase, has been added.

### ASSAYS AND DEVICES

The term "assay device" as used herein means an apparatus, collection of apparatuses or equipment.

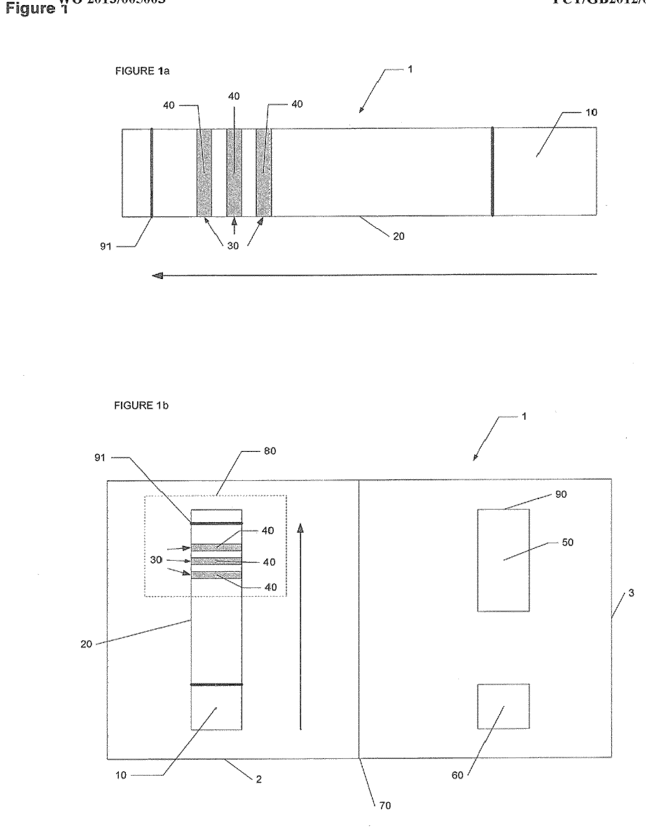

An assay device or apparatus comprising the features listed in (a) to (e) above is shown in Figure 1.

With reference to Figure 1, the assay device (1) of the present invention is shown as two embodiments - presented in Figure 1(a) (embodiment 1) and in Figure 1(b) (embodiment 2).

In both embodiments, the assay device (1) comprises a placement region (10) operably connected to matrix (20). At least one distinct capture location (30) is present on the matrix (20). Capture means (40) are present at or define each distinct capture location (30). In use, the operator places the sample on the placement region (10) and the sample migrates along the matrix (20) in the direction shown by arrows. In embodiment 1, selective indication means (50) (not shown), or at least a component thereof (not shown), are added to or are constituted at the capture location (30). In one aspect, the selective indication means comprises a reagent (reactive species) that can generate an observable colour change when the active enzyme has reacted with a substrate. In that aspect, the reagent can be supplied in liquid form to the device in use.

Embodiment 2 of the assay device (1) functions in the same way as embodiment 1. However, in this case the assay device (1) consists of two opposing halves (2) and (3) which are joined by folding section or fold line (70). The placement region (10), operably connected to the matrix (20) is placed in one half (2) of the device. The capture location/s (30) and capture means (40) are placed on the matrix (20). The same or opposite half (3) of the device comprises the selective indication means or at least a component thereof, and the opposite half (3) optionally comprises an absorbent pad to soak up excess sample (60).

The selective indication means (50), or at least a component thereof, of embodiment 2 is brought into contact with the matrix (20) and capture means (40) when the operator folds fold line (70). The operator may need to add the selective indication means (50) or some component/s of the selective indication means (50) to the device in liquid form. Alternatively the selective indication means or at least some components of the selective indication means may be present in dried form in the position (90).

Optionally preferred, embodiment 2 comprises viewing window (80) which allows the operator to view the result of the assay device (in other words, view the capture locations) once the two halves (2) and (3) of the device are closed. The viewing window (80) may be made of a plastics material or it may be an open aperture.

In embodiment 2, one or each of the halves (2, 3) can be made from plastics or card materials, sufficiently stiff enough to provide support.

As mentioned, the assay device (1) comprises a placement region where a sample can be placed. Preferably the sample is a liquid sample. The matrix (20) is operably connected to the placement region (10) such that the sample can migrate from the placement region (10) along the matrix (20). Preferably the matrix (20) is a material which allows the sample to migrate, such as e.g. nitrocellulose. The matrix (20) of the assay device (1) of the current application comprises at least one distinct capture location (30) which is distanced away from the placement region (10). The sample can migrate across the capture location (30). Preferably three capture locations (30) are used. At each capture location (30) in the assay device (1) of the current invention, capture means (40) are present. The capture means (40) are capable of binding to the enzyme to be detected by the assay device (1). At least a sample of the enzyme is retained at at least one capture location (30). The capture means (40) are preferably antibodies and most preferably the capture means (40) are antibodies that bind to the enzyme. The antibodies may be raised against the enzyme *per se* or against a component thereof (e.g. a fragment thereof and/or an epitope thereof).

Selective indication means (50) are used in the assay device (1) of the current invention to provide selective indication of the presence of active enzyme bound to the capture means (40). Preferably the selective indication means or at least a component thereof is in position (90) on the opposing half of the assay device (1) compared to the matrix (20). Preferably the selective indication (50) means comprises a precipitation reaction and/or provides a visible colour change. The colour change may be caused by the reaction of a substrate with the enzyme. Reaction with a substrate indicates the enzyme is active and not merely present in an inactive or de-natured form.

In a preferred aspect, the device is semi-quantitative - in the sense that the amount of active enzyme (analyte quantity) is indicated by the number of capture locations (30) of the device which display a colour change and/or by the intensity of the colour change at the capture location(s) if the selective indication means generates a colour change when active enzyme is present.

The assay device (1) of the current invention preferably comprises several regions which are operably connected. The regions preferably include a placement region (10). Most preferably the assay device (1) of the current invention is a lateral flow device.

The assay device (1) of the current invention may optionally comprise an "indicator region" or "indicator line" (91) which indicates if the assay device has worked correctly. Preferably the indicator region or line indicates the assay has worked through a colour or colour change. The optional indicator region or line may comprise antibodies. These antibodies may be raised against an enzyme *per se* or against a component thereof (e.g. a fragment thereof and/or an epitope thereof) wherein said enzyme is unrelated to the active enzyme to be detected. In addition or alternatively the indicator line may comprise a related or unrelated enzyme which can react with at least some of the substrate components. In one embodiment of the invention, the device and assay are used to detect active phytase enzyme. In such a case, the related or unrelated enzyme at the indicator line can be an enzyme having phosphatase activity. By way of example, the indicator line may comprise a related enzyme or the same enzyme as the one to be detected, e.g. the indicator line may comprises BP17 phytase in an assay for detecting BP17 phytase. By way of a further example, the indicator line may comprise an enzyme and an antibody, wherein the antibody may be specific to the enzyme of the indicator line or another enzyme.

An "assay" is the analysis of the chemical composition or strength of a substance. This can include detection of a particular constituent in a mixture. The assay device (1) is used to perform the assay of the invention, which is to detect active enzyme.

The terms "immunoassay" and "assay" are used interchangeably in this application wherein the term "assay" can encompass "immunoassay". An immunoassay is a particular form of assay which uses the specific binding between an antigen and its antibody to identify a substance in a sample.

The assay device may preferably comprise a blocking reagent, or a blocking reagent may be added to the device.

The term "blocking reagent" refers to an agent which modulates the interaction of the active enzyme to be detected (i.e. the analytes) with the capture means, preferably wherein the capture means comprises antibodies. The blocking reagent allows movement of the sample along the matrix of the device so that the entire sample is not blocked by the first capture means. The blocking reagent may be or may comprise a surfactant. Examples of suitable blocking reagents or components of blocking reagents include casein and Dehquart CC6

The matrix (20) may comprise the blocking reagent. Alternatively the blocking reagent or a component thereof may be added to the device, The blocking reagent may be added at or near to or even away from components (30) of the assay device. The blocking reagent may be bound to the matrix (20) or applied on a pad to matrix (20). The blocking agent may be in a dried form and/or a dehydrated form.

### ENZYMES

The term "enzyme" as used herein refers to a protein which catalyses the chemical reactions of other substances without itself being destroyed or altered upon completion of the reactions.

The enzyme detected by the current invention can be a wild-type, which is an enzyme present in nature, or a variant. A "variant" is an enzyme having an amino acid sequence which has one or several insertions, deletions and/or substitutions compared with the parent sequence from which the variant is derived of such as a wild-type enzyme or even a variant enzyme, and which retains a functional property and/or enhances a property, e.g. an enhanced activity, of the enzyme. As used herein, the term "amino acid sequence" is synonymous with the term "polypeptide" and/or the term "protein".

The term "active enzyme" as used herein refers to an enzyme which retains its catalytic function. For example, phytase is capable of catalysing the hydrolysis of esters of phosphoric acid.

The term "inactive enzyme" refers to enzyme which is present in a sample but is incapable of performing its catalytic function. Inactivation may occur due to denaturation of the enzyme, due to heat treatment or due to chemical treatment or treatment or processing by another enzyme such as proteolysis by a protease or deglycosylation by a glycosidase. Inactivation may also be due to a chemical inhibitor. In the case where the enzyme is phytase, such as e.g. phytase BP17, an inhibitor which can be used is Myoinositol Hexasulphate (MIHS).

Examples of enzymes detected by the current invention include phosphatases and glycoside hydrolases.

Phosphatases or phosphoric monoester hydrolases (EC 3.1.3) are enzymes able to release phosphate groups from their substrate. Useful phosphatases include, but are not limited to, alkaline phosphatase (EC 3.1.3.1), acidic/acid phosphatase (EC3.1.3.2), 3-phytase (EC 3.1.3.8) or 6-phytase (EC 3.1.3.26). Preferably the enzyme detected by the assay device of the current invention is a phytase. Preferably the enzyme is 6-phytase and most preferably the enzyme is 6-phytase (BP17) derived from *Buttiauxella sp.*

The 6-phytase is also called "4-phytase" or "phytate 6-phosphatase". Further phytases include histidine acid phytases (HAP), which is a group comprising members found among prokaryotes (e.g. appA phytase from *Escherichia coli*) and eukaryotes (phyA and B from *Aspergillus* sp., HAP phytases from yeast and plants. HAP phytases share a common active site motif, RHGXRXP, at the N-terminal end and a HD motif at the C-terminal end in their DNA sequences. This allows a two-step mechanism in the hydrolysis of phosphomonoesters. The phyA and phyB from A. *niger* and appA from *E. coli* are the representatives which have been the most characterized.

In a preferred aspect, the enzyme is a phytase and active enzyme activity is determined by reduction of a reactive species - such as a tetrazolium salt - to produce a visually observable effect, such as a visually observable precipitate which may be coloured. The device is semi-quantitative and analyte quantity is indicated by the number of capture locations (30) of the device which display a colour change due to the precipitate. The tetrazolium reduction that produces colour is in essence an indirect measure of the phosphatase enzyme action on its own substrate. In this respect, a reducing agent or reactive moiety is generated that then acts on the tetrazolium to generate formazan dye.

In one embodiment the enzyme detected by the current invention is a 6-phytase from e.g. *Buttiauxella* sp. *Trichoderma reesei, E. coli, Aspergillus niger, Citrobacter braakii, Citrobacter freundii, Peniphora lycii or Penicillium funiculosum*

In one embodiment the phytase is a *Citrobacter* phytase derived from e.g. *Citrobacter freundii,* preferably *C*. *freundii* NCIMB 41247 and variants thereof e.g. as disclosed in WO2006/038062 (incorporated herein by reference) and WO2006/038128 (incorporated herein by reference), *Citrobacter braakii* YH-15 as disclosed in WO 2004/085638, *Citrobacter braakii* ATCC 51113 as disclosed in WO2006/037328 (incorporated herein by reference), as well as variants thereof e.g. as disclosed in WO2007/112739 (incorporated herein by reference) and WO2011/117396 (incorporated herein by reference), *Citrobacter amalonaticus,* preferably *Citrobacter amalonaticus* ATCC 25405 or *Citrobacter amalonaticus* ATCC 25407 as disclosed in WO2006037327 (incorporated herein by reference), *Citrobacter gillenii,* preferably *Citrobacter gillenii* DSM 13694 as disclosed in WO2006037327 (incorporated herein by reference), or *Citrobacter intermedius, Citrobacter koseri, Citrobacter murliniae, Citrobacter rodentium, Citrobacter sedlakii, Citrobacter werkmanii, Citrobacter youngae, Citrobacter* species polypeptides or variants thereof.

In one embodiment the phytase may be a phytase from *Citrobacter,* e.g. from *Citrobacter freundii,* such as the phytase enzyme(s) taught in WO2006/038128, which reference is incorporated herein by reference.

In some embodiments, the phytase is preferably *E. coli* phytase marketed under the name Phyzyme XP™ by DuPont Nutrition Biosciences ApS.

Alternatively the phytase may be a *Buttiauxella* phytase, e.g. a *Buttiauxella agrestis* phytase, for example, the phytase enzymes taught in WO 2006/043178, WO 2008/097619, WO09/129489, WO2008/092901, or WO10/122532, all of which are incorporated herein by reference.

In one embodiment the phytase may be a phytase from *Hafnia,* e.g. from *Hafnia alvei,* such as the phytase enzyme(s) taught in US2008263688, which reference is incorporated herein by reference.

In one embodiment the phytase may be a phytase from *Aspergillus,* e.g. from *Apergillus orzyae.*

In one embodiment the phytase may be a phytase from *Penicillium,* e.g. from *Penicillium funiculosum.*

It will be understood that as used herein 1 FTU (phytase unit) is defined as the amount of enzyme required to release 1 µmol of inorganic orthophosphate from a substrate in one minute under the reaction conditions defined in the ISO 2009 phytase assay - A standard assay for determining phytase activity and 1 FTU can be found at *International Standard ISO*/*DIS 30024: 1-17, 2009.*

Alternatively, as used herein one unit of phytase (FTU) is defined as the quantity of enzyme that releases 1 micromol of inorganic phosphorus/min from 0.00015 mol/L of sodium phytate at pH 5.5 at 37 degrees C (Denbow, L. M., V. Ravindran, E. T. Kornegay, Z. Yi, and R. M. Hulet. 1995. Improving phosphorus availability in soybean meal for broilers by supplemental phytase. Poult. Sci.74:1831-1842)."

In one embodiment suitably the enzyme is classified using the E.C. classification above, and the E.C. classification designates an enzyme having that activity when tested in the assay taught herein for determining 1 FTU.

Another group of enzymes which may be detected by the assay device and method of the current invention is the group of glycoside hydrolases (EC 3.2.1), i.e. enzymes hydrolysing O- and S-glycosyl compounds. A further common property of this group of enzymes is that they release for instance a fragment or product having a reducing end-group (containing aldehyde group). Examples of this group includes xylanases, such as endo-1,4-β-xylanase (EC 3.2.1.8) and xylan endo-1,3-β-xylosidase (EC 3.2.1.32), preferable xylan 1,4-beta-xylosidase (EC 3.2.1.37); α-amylase (EC 3.2.1.1); β-amylase (EC 3.2.1.2); oligo-1,6-glucosidase (EC 3.2.1.10); glucan 1,4-alpha-glucosidase (EC 3.2.1.3); pullulanase (EC 3.2.1.41); cellulase (EC 3.2.1.4); endo-1,3(4)-beta-glucanase (EC 3.2.1.6); or glucan endo-1,3-beta-D-glucosidase (EC 3.2.1.39).

Xylanase is the name given to a class of enzymes which degrade the linear polysaccharide beta-1,4-xylan into xylose, thus breaking down hemicellulose, one of the major components of plant cell walls.

The xylanase for use in the present invention may be any commercially available xylanase.

Suitably the xylanase may be an endo-1,4-β-d-xylanase (classified as E.C. 3.2.1.8) or a 1,4 β-xylosidase (classified as E.C. 3.2.1.37).

In one embodiment preferably the xylanase in an endoxylanase, e.g. an endo-1,4-β-d-xylanase. The classification for an endo-1,4-β-d-xylanase is E.C. 3.2.1.8.

Amylases may also be detected by the assay device and method of the current invention.

Amylase is the name given to a class of enzymes capable of hydrolysing starch to shorter-chain oligosaccharides such as maltose. The glucose moiety can then be more easily transferred from maltose to a monoglyceride or glycosylmonoglyceride than from the original starch molecule.

The term amylase includes α-amylases (E.C. 3.2.1.1), G4-forming amylases (E.C. 3.2.1.60), β-amylases (E.C. 3.2.1.2) and γ-amylases (E.C. 3.2.1.3).

In one embodiment preferably the amylase is an α-amylase. α-Amylases are classified as (E.C. 3.2.1.1).

Preferably the substrate is a phosphatase substrate and most preferably the phosphatase substrate is 2-phosph-L-ascorbic acid trisodium (AsAP).

### SAMPLE

The term "sample" as used herein means a specimen or extraction of the substance or composition collected for analysis to determine whether active enzyme is present.

The sample is preferably a liquid. The sample is preferably obtained by placing, dissolving, liquefying or mashing the substance or composition to be analysed in a solvent, such as water. Preferably the sample is an aqueous sample. Tap water or purified and deionised water such a MilliQ or another suitable aqueous solution may be used. It is envisaged that to prepare the sample some form of extraction may be needed to allow the substance or composition to be analysed to mix with, break down and/or dissolve in the solvent. For example, agitation may be used.

It is envisaged that a kit will be produced wherein the operator will fill a sample extraction container (e.g. a tube, plastic bag or cannister) to a predetermined level (marked by a line on the container) with food or feed or biofuel or detergent composition and then fill the tube to a second line with water such as tap water, purified or deionised water. Subsequently the extraction container is preferably shaken. This will enable the operator to make the sample in a uniform manner.

The sample of the present invention may be derived, extracted or taken from a food or feed, or from a food or feed composition, or from a detergent or from a detergent composition or from ethanol or biofuel production. Here, the term "food" is used in a broad sense - and covers food for humans as well as food for animals (i.e. a feed). In a preferred aspect, the food is for human consumption. In another preferred aspect, the food is a non-human animal feed.

The food or feed may be in the form of a solution, or slurry, or as a solid such as a pellet depending on the use and/or the mode of application and/or the mode of administration.

The terms "food" and "feed" may also refer to a food ingredient. The sample of the present invention may be derived, extracted or taken from a food ingredient.

As used herein the term "food or feed ingredient" includes a formulation which is or can be added to functional foods or foodstuffs as a nutritional supplement and/or fibre supplement. The term "food or feed ingredient" as used here also refers to formulations which can be used at low levels in a wide variety of products to give beneficial functional effects relevant for the particular feed or foodstuff desired, including but not limited to gelling, texturising, stabilising, emulsifying, suspending, film-forming and structuring, retention of juiciness and improved mouthfeel, without adding viscosity.

In a further embodiment, "food or feed ingredient" includes food intermediates such as bread improver compositions or dough prior to baking. In the bakery application area, enzymes are typically added to dough compositions prior to baking, and in preferred embodiments the denaturation of the enzymes during the baking process terminates further enzyme activity. Therefore, the enzyme assay of the current invention must be performed in the dough or dough ingredient composition prior to baking. In a further embodiment, the current invention can be used in dairy food compositions containing cereal components, preferably oats, rye, rice, wheat, wheat bran, or processed derivatives thereof. Preferably said dairy food composition is a yoghurt, cheese, or culture drink.

The food ingredient may be in the form of a solution, or slurry, or as a solid such as a pellet depending on the use and/or the mode of application and/or the mode of administration.

The terms "food" and "feed" may also refer to a food additive. The sample of the present invention may be derived, extracted or taken from a food additive.

As used herein, the term "food or feed additive" includes formulations which enhance the digestion in food and animal feeds. In particular "food or feed additive" relates to phytases which can be used for enhancing phosphate digestion in foods and animal feeds.

The food additive may be in the form of a solution, or slurry, or as a solid such as a pellet depending on the use and/or the mode of application and/or the mode of administration.

The feed composition, and/or feed additive may comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

The present invention is useful for detecting active enzyme in biofuel production, such as bioethanol production.

### STRUCTURE OF ASSAY DEVICE

The assay device (1) of the current invention comprises a placement region (10). The term "placement region" as used here in to refer to a region of the assay device (1) onto which the sample can be placed. Preferably the placement region (10) is an absorbent pad. The placement region (10) is operably connected to a matrix (20). The placement region (10) may be contiguous or continuous with the matrix (20). The placement region (10) can be an integral region of the matrix (20). Preferably the placement region (10) is at one end of the matrix (20). Optionally an additional absorbent pad is present to soak up excess sample (60).

In the context of the current description "operably connected" means joined, fastened together or in contact with during operation of the device.

Preferably the matrix (20) is or comprises an absorbent material. More preferably the matrix (20) is or comprises nitrocellulose. For example the matrix (20) may be or may comprise nitrocellulose of type MDI 90 CNPH, MDI SS12 12µ or SS12 15µ.

When present or placed on the sample region (10), the sample can migrate along the matrix (20). Preferably the migration is liquid migration such as capillary action. Preferably the migration is in a longitudinal direction, and most preferably this occurs when the assay device (1) is a strip or stick. Preferably the stick or strip is contained in a housing.

A blocking reagent may be added to the assay device. The matrix (20) may comprise the blocking reagent. The blocking reagent may be bound to the matrix (20) and the blocking agent may be in a dried or dehydrated form. Here, "bound" includes coupled or attached. The blocking reagent may be directly or indirectly bound to the matrix. The blocking reagent may be any suitable blocking reagent. An example of a blocking reagent is casein. The blocking reagent may be or may comprise surfactant. The blocking reagent preferably comprises a surfactant. The blocking reagent is preferably a non-ionic surfactant and/or a Zwitterionic surfactant. For example the surfactant may be Tween-20 or Dehquart CC6.

The assay device (1) of the current invention comprises at least one distinct capture location (30) on the matrix (20). Each capture location (30) is distanced away from the placement region (10) and the sample can migrate across the distinct capture location/s (30). "Distinct" as used herein in this context means separated from other capture locations (30).

The assay device (1) of the current invention may comprise multiple distinct capture locations (30); preferably more than two distinct capture locations (30) and most preferably the device comprises three distinct capture locations (30).

The assay device (1) may have two or more capture location regions, each region comprising one or more discrete capture locations (30). For example, when having three discrete capture locations regions it may be possible to capture at least three different types of enzymes, such as e.g. phytase, xylanase and amylase in one sample and one measurement.

At each capture location (30) there are capture means (40). The capture means (40) at each capture location (30) may be different or may be the same. The capture means (40) are capable of binding to the enzyme which is to be detected by the assay device (1). This results in at least a sample or part of the sample of the enzyme being retained at at least one distinct capture location (30). The binding is between the capture means (40) and the enzyme is preferably the result of antibody/antigen binding. The capture means (40) alone may define the capture location (30).

The amount of capture means (40) at each distinct capture location (30) of the assay device (1) may be different or the same. The amount of capture means (40) at each distinct capture location (30) may increase the further the location is away from the placement region (10). The amount of capture means (40) at each distinct capture location (30) may decrease the further the location is away from the placement region (10).

In some embodiments, the amount of capture means (40) at each distinct capture location (30) of the assay device (1) is substantially the same.

The assay device (1) of the current invention may optionally comprise an indicator region or line (91) which indicates if the assay device has worked correctly. Preferably the indicator region or line indicates the assay has worked through a colour change. The optional indicator region or line may comprise antibodies. These antibodies may be raised against an enzyme *per se* or against a component thereof (e.g. a fragment thereof and/or an epitope thereof) wherein said enzyme is unrelated to the active enzyme to be detected. In addition or alternatively the indicator line may comprise an unrelated enzyme which can react with at least some of the substrate components.

### ANTIBODIES

The capture means (40) of the present invention are preferably antibodies that bind to the active enzyme to be detected by the assay device (1). The antibodies may be raised against the enzyme *per se* or against a component thereof (e.g. a fragment thereof and/or an epitope thereof).

The optional indicator region or line (91) of the assay device (1) of the current invention may comprise antibodies.

Most preferably the capture means (40) are antibodies raised against a phytase or a component thereof (e.g. a fragment thereof and/or an epitope thereof), such as phytases BP17, BP11 or BP111.

Antibodies may be produced by standard techniques, such as by immunisation immunization with the enzyme or interest, as a mixture, purified sample or a fragment thereof or by using a phage display library.

For the purposes of this invention, the term "antibody", unless specified to the contrary, includes, but is not limited to, polyclonal, monoclonal, chimeric, single chain, Fab fragments, fragments produced by a Fab expression library, as well as mimetics thereof. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. Furthermore, the antibodies and fragments thereof may be humanised antibodies.

Neutralising antibodies (i.e. those which inhibit biological activity of the substance polypeptides and which are commonly used in therapeutics) are not preferred for the device or assay of the present invention.

If polyclonal antibodies are desired, a selected animal (e.g., mouse, rabbit, goat, horse, chicken, etc.) is immunised with the enzyme to be detected (or a polypeptide fragment comprising an immunological epitope thereof). Depending on the host species, various adjuvants may be used to increase immunological response.

Serum from the immunised animal is collected and treated according to known procedures. If serum containing polyclonal antibodies to the enzyme to be detected by the assay device of the current invention, and/or amino acid sequence of that enzyme (or a sequence comprising an immunological epitope thereof) contains antibodies to other antigens, the polyclonal antibodies can be purified by immunoaffinity chromatography. Techniques for producing and processing polyclonal antisera are well known in the art (e.g. Harboe N, Ingild A. Immunization, isolation of immunoglobulins, Estimation of antibody titre. Scand J Immunol Suppl. 1973, 1:161-4). In the Example section, the methodology of Harboe (*ibid*) was used to generate antibodies to the phytase enzyme BP17. However, other techniques could be used and/or other enzymes could be used to generate suitable antibodies.

Monoclonal antibodies directed against the enzyme to be detected by the assay device (1) of the current invention, and/or amino acid sequence of that enzyme (or a sequence comprising an immunological epitope thereof) can also be readily produced by one skilled in the art and include, but are not limited to, the hybridoma technique Koehler and Milstein (1975 Nature 256:495-497), the human B-cell hybridoma technique (Kosbor et al., (1983) Immunol Today 4:72; Cote et al., (1983) Proc Natl Acad Sci 80:2026-2030) and the EBV-hybridoma technique (Cole et al., (1985) Monoclonal Antibodies and Cancer Therapy, Alan Rickman Liss Inc, pp 77-96).

In addition, techniques developed for the production of "chimeric antibodies", the splicing of mouse antibody genes to human antibody genes to obtain a molecule with appropriate antigen specificity and biological activity may be used (Morrison et al., (1984) Proc Natl Acad Sci 81:6851-6855; Neuberger et al., (1984) Nature 312:604-608; Takeda et al., (1985) Nature 314:452-454).

Alternatively, techniques described for the production of single chain antibodies (US4946779) can be adapted to produce the substance specific single chain antibodies.

Antibody fragments which contain specific binding sites for the substance may also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed to allow rapid and easy identification of monoclonal Fab fragments with the desired specificity (Huse WD et al., (1989) Science 256:1275-128 1).

In some embodiments, preferably the antibodies are polyclonal antibodies.

The antibodies may be modified - e.g. derivatised - to suit even better the device or the assay. For example, the antibody may be coupled with one or more chemical moieties that allow for specific attachment/coupling to matrices such as derivatised plastics.

### SELECTIVE INDICATION MEANS

The assay device of the current invention comprises a selective indication means (50) or at least a component thereof to provide selective indication of the presence of active enzyme bound to the capture means (40). The selective indication means (50) may comprise a precipitation reaction.

In one aspect, the assay device comprises all or substantially all of the selective indication means.

In another aspect, the assay device is provided with at least one component of the selective indication means. In that case, the assay device is provided with the other component(s) of the selective indication means. In one aspect, the selective indication means are constituted on and/or in the assay device when the assay device is in use. The term "constituted" includes mixing or contacting of the components to generate the selective indication means.

In another aspect, a kit is provided wherein a device is provided that can be used as the assay device of the present invention and wherein the kit is provided with the selective indication means. In use, all or some of the components of the selective indication means then added to the device so as to form the assay device of the present invention.

The term "provide/s/d" as used herein refers to being capable of delivering the indication on its own or as part of a system. Alternatively the term "provide/s/d" or "provided" refers to a kit or device containing or comprising the identified component.

The selective indication means (50) may comprise or provide a visible colour change. The colour change is preferably caused by reaction of a substrate of the enzyme to form a moiety that reacts with a reactive species. The substrate may be bound to the matrix (20).

The material on which an enzyme acts is referred to as its "substrate". Preferably the substrate of the enzyme is bound to the assay device (1). Here, "bound" includes coupled or attached. The substrate may be directly or indirectly bound to the device. Most preferably the substrate is bound to the matrix (20) of the assay device (1), or it may be present on the opposite half (3) of the device in position (50). The substrate is preferably in a dried form.

Preferably the enzyme acts on its substrate to generate a reactive moiety. Said reactive moiety can then act on a reactive species to generate a functional effect, preferably a visually observable effect such as a colour change.

Said reactive moiety may also be referred to herein as a moiety, a reducing moiety, reducing species or a reducing agent.

The substrate may be a phosphatase substrate or a compound with a phosphate donor group. Preferably, the substrate is an acid phosphatase substrate. For some aspects, the removal of the phosphate donor group yields a reducing agent or reactive moiety. Preferably the phosphatase substrate is 2-phospho-L-ascorbic acid trisodium (AsAP). Other examples, include analogs, such as alternative ascorbyl 2-phosphate salts (e.g. magnesium, zinc, calcium) or the 3-O-phosphate derivative. Other possible examples include esterified versions of ascorbyl phosphate (phospho ascorbyl palmitate).

When the selective indication means (50) comprises a colour change caused by reaction of a substrate of the enzyme to form a moiety that reacts with a reactive species, the reactive species may be a tetrazolium compound. In this respect, the colour change is caused by the formation of a formazan dye as a result of the enzyme reaction.

Tetrazolium salts have been used in industry to remove substances which would disturb the colour change of a redox reaction. For example, see US 5,196,314. In addition, the colour change of a tetrazolium salt has been used as an indicator of the presence of an analyte in US 5,360,595.

In the present invention, the reactive species may be a nitroblue tetrazolium compound. The reactive species may be nitroblue tetrazolium chloride (NBT). The reactive species may alternatively be a halonitrotetrazolium compound. The reactive species may preferably be an iodonitrotetrazolium compound. Preferably the reactive species is iodonitrotetrazolium chloride (INT).

To the selective indication means, and most preferably to the reactive species, may be added an entity capable of reducing or preventing diffusion of the precipitate. This improves the quality of the result, and most preferably improves the visual lines of the precipitate. Most preferably this entity is polyethylene glycol and most preferably this is added to the tetrazolium salts.

Thus, in a preferred aspect, there is described a lateral flow assay device (1) which detects active enzyme in a sample. Preferably enzyme is an acid phosphatase and enzyme activity is determined by reduction of a tetrazolium salt to produce a coloured precipitate. The device is semi-quantitative and analyte quantity is indicated by the number of capture locations (30) of the device which display a colour change due to the precipitate.

When the selective indication means (50) comprises a phosphatase substrate as described above, the phosphatase substrate may be reacted with the reactive species, as described above, in the presence of a reaction enhancer. The reaction enhancer may be bound to a secondary matrix. The reaction enhancer may be a phenazine compound.

Preferably the reaction enhancer is a methosulphate compound. Most preferably the reaction enhancer is phenazine methosulphate (PMS).

In preferred embodiments the active enzyme to be detected acts on the substrate to generate a reactive moiety or reducing agent that then acts on an entity that produces an observable effect.

In a preferred embodiment the enzyme is phytase, the substrate is phosphatase substrate or a compound with a phosphate donor group.

In another preferred embodiment the enzyme is xylanase, the substrate is xylan and the reactive moiety is xylose.

In another preferred embodiment the enzyme is amylase, the substrate is starch and the reactive moiety is glucose.

In a preferred embodiment the entity is a tetrazolium salt and the observable effect is a visible effect, most preferably a colour change..

### PREFERRED EMBODIMENTS

Two preferred embodiments of the assay device (1) of the current invention are shown in Figure 1a and Figure 1b.

Preferably the assay device (1) is a strip or stick. Along this strip or stick a liquid sample can flow longitudinally in the direction shown by arrows in Figure 1. The strip or stick body comprises the matrix (20). As in embodiment 1 of the device (Figure 1a), the placement region (10) is preferably at one end of the strip or stick. The capture locations (30) are preferably at the opposite end of the strip or stick to the placement region (10).

The selective indication means (50) or a component thereof may be added to the assay device when in use. Preferably these indication means or at least a component thereof are in liquid form. Preferably all of the reagents are dried except the selective indication means (50) or some component of the selective indication means (50). Alternatively all of the reagents may be in a dried or dehydrated form.

Preferred embodiment 2 of the assay device (1) is shown in Figure 1b. In this case the assay device (1) consists of two opposing halves which are connected by a folding section or fold line (70). The placement region (10), operably connected to the matrix (20) are placed in one half (2) of the device. The capture location/s (30) and capture means (40) are placed on the matrix (20). The same (2) or opposite half (3) of the device comprises the selective indication means, or at least a component thereof, and optionally the opposite half (3) comprises an absorbent pad to soak up excess sample (60).

When using embodiment 2, the operator places the sample on the placement region (10). He/she then allows the sample to flow to at least a required position. Then he/she closes the device along the folding section (70). This brings the selective indication means (50), or at least a component thereof, into contact with the matrix (20) and capture means (40). The operator may need to add the selective indication means (50) or some component/s of the selective indication means (50) to the device in liquid form.

For some embodiments, the selective indication means or some components of the selective indication means may be present in dried form in the position (90). Preferably all of the reagents are dried. In some embodiments a tetrazolium solution of either INT or NBT may be added by the operator to position (50) or (30).

Optionally preferred, embodiment 2 comprises viewing window (80) which allows the operator to view the result of the assay device (in other words, view the capture locations) once the two halves (2) and (3) of the device are closed.

The assay device (1) of the current invention may optionally comprise an indicator region or line which indicates if the assay device has worked correctly. Preferably the indicator region or line indicates the assay has worked through a colour change. The optional indicator region or line may comprise antibodies. These antibodies may be raised against an enzyme *per se* or against a component thereof (e.g. a fragment thereof and/or an epitope thereof) wherein said enzyme is unrelated to the active enzyme to be detected. In addition or alternatively the indicator line may comprise an unrelated enzyme which can react with at least some of the substrate components.

In addition or in the alternative the indicator region may comprise an acid phosphatase or phytase unrelated to the phytase to be detected.

In one embodiment, the present invention provides an assay device for detecting active enzyme in a sample; wherein the device comprises: (a) a placement region onto which the sample can be placed; (b) a matrix operably connected to said placement region such that the sample when present (such as placed) on said placement region can migrate along said matrix; (c) at least one distinct capture location on said matrix, wherein each distinct capture location is distanced away from the placement region, and wherein the sample can migrate across said distinct capture location; (d) capture means being present at or defining each distinct capture location, wherein said capture means are capable of binding to said enzyme such that at least a portion of said sample of said enzyme is retained at at least one distinct capture location; and (e) selective indication means or at least a component thereof to provide selective indication of the presence of active enzyme bound to said capture means.

Preferably, the enzyme is phytase.

In one aspect, the present invention provides an assay device for detecting active enzyme in a sample; wherein the device in use comprises: (a) a placement region onto which the sample can be placed; (b) a matrix operably connected to said placement region such that the sample when present (such as placed) on said placement region can migrate along said matrix; (c) at least one distinct capture location on said matrix, wherein each distinct capture location is distanced away from the placement region, and wherein the sample can migrate across said distinct capture location; (d) capture means being present at or defining each distinct capture location, wherein said capture means are capable of binding to said enzyme such that at least a portion of said sample of said enzyme is retained at at least one distinct capture location; and (e) selective indication means to provide selective indication of the presence of active enzyme bound to said capture means.

Preferably, the enzyme is phytase.

In one embodiment, the present invention provides an assay device for detecting active enzyme in a sample; wherein the device comprises: (a) a placement region onto which the sample can be placed; (b) a matrix operably connected to said placement region such that the sample when present (such as placed) on said placement region can migrate along said matrix; (c) at least one distinct capture location on said matrix, wherein each distinct capture location is distanced away from the placement region, and wherein the sample can migrate across said distinct capture location; (d) capture means being present at or defining each distinct capture location, wherein said capture means are capable of binding to said enzyme such that at least a portion of said sample of said enzyme is retained at at least one distinct capture location; and (e) selective indication means or at least a component thereof to provide selective indication of the presence of active enzyme bound to said capture means; and wherein the enzyme is a phytase and wherein the selective indication means comprises an acid phosphatase.

### SEMI-QUANTITATIVE

The assay device (1) of the current invention may provide at least a semi-quantitative measure of the amount of active enzyme in a sample.

Preferably the quantity is relative to the number of capture locations (30) which indicate the presence of bound enzyme. Alternatively, semi-quantitative measurement can be made using the intensity of a colour change at the capture location/s (30).

The intensity of a colour change may be evaluated using a score card or reference card, preferably wherein the operator compares the intensity of the colour reaction on the matrix (20) to the score card. In addition, or alternatively, the user may use an electronic device that can read the colour change and report back to the user the result(s) of its analysis. By way of example, the electronic device may be a mobile telephone that may comprise a suitable "app".

The semi-quantitative measurement may determine whether the amount of active enzyme is above or below a certain amount or concentration, for example above or below 300 FTU/g.

### METHODS AND USES OF THE INVENTION

As described above, the current invention provides a method or use (e.g. using the device of the present invention) to determine if there is active enzyme present in a sample. Preferably this method is semi-quantitative. The method involves using the assay device (1) described herein with a sample, and comparing the results to other samples.

A sample that contains more active enzyme causes more capture locations (30) to indicate the presence of bound active enzyme.

Alternatively or in addition a sample which causes a stronger (e.g. darker) colour change at capture locations contains more active enzyme.

Preferably the method is used to determine active phosphatases or phosphoric monoester hydrolases (EC 3.1.3) and glycoside hydrolases (EC 3.2.1), including alkaline phosphatase (EC 3.1.3.1), acidic/acid phosphatase (EC3.1.3.2), 3-phytase (EC 3.1.3.8), 6-phytase (EC 3.1.3.26), endo-1,4-β-xylanase (EC 3.2.1.8) and xylan endo-1,3-β-xylosidase (EC 3.2.1.32), preferable xylan 1,4-beta-xylosidase (EC 3.2.1.37); α-amylase (EC 3.2.1.1), β-amylase (EC 3.2.1.2), oligo-1,6-glucosidase (EC 3.2.1.10), glucan 1,4-alpha-glucosidase (EC 3.2.1.3), pullulanase (EC 3.2.1.41), cellulase (EC 3.2.1.4), endo-1,3(4)-beta-glucanase (EC 3.2.1.6), and glucan endo-1,3-beta-D-glucosidase (EC 3.2.1.39). More preferably active 6-phytase, such as phytase BP17, levels are determined.

A further aspect of this invention is it provides a method of determining levels of active enzyme in a food or feed or biofuel or detergent composition. This method involves preparing a sample of the food or feed or biofuel or detergent composition, preferably an aqueous sample. Then the sample is placed on the placement region (10) of the assay device (1) of the invention, and the assay device (1) is used to determine the amount of active enzyme in the sample semi-quantitatively. The amount of active enzyme in several samples can be compared to each other and/or compared to a reference sample. Most preferably this method is used to determine active phytase levels, preferably active phytase BP17 levels, in a food or feed.

The current invention further provides a method of determining whether additional enzyme or a feed or food additive is needed. If less than a specified quantity or relative quantity of active enzyme is detected using the method described above, this indicates that additional enzyme should be added. Most preferably this method is used to determine whether additional phytase, preferably active phytase BP17, should be added to a food or feed.

The methods described above can be used for quality control. Such a method comprises using the assay device (1) of the current invention to determine active enzyme levels in a sample and then determining if these enzymes meet the quality control level of enzyme. Preferably such a method could be used for determining phytase levels in a feed or a feed additive and then determining if the feed or a feed additive is to be used

The current invention further provides a method of determining the presence of active enzyme in a sample wherein the method comprises the steps of:
a) contacting at least a portion of the sample with a capture means whereby the enzyme is captured by said capture means;
b) providing a suitable substrate for the captured enzyme wherein said substrate reacts with said captured enzyme to yield an enzyme product;
c) providing a reactive species capable of reacting with the enzyme product wherein said reactive species reacts with said enzyme product to yield an insoluble product; and
d) detecting the insoluble product obtained in step c) wherein said detecting correlates to the presence and/or quantity of an active enzyme in said sample.

Preferably the sample is a food or feed sample or food or feed component sampleor biofuel or detergent composition sample. Preferably the sample is dried, re-hydrated or an aqueous sample. Most preferably the sample is a liquid sample.

Preferably the active enzyme detected by the method is a phosphatase or glycoside hydrolase, most preferably an acid phosphatase, a phytase, a xylanase, an amylase, or a β-glucanase.

Preferably the capture means used in the method are antibodies that bind to the active enzyme to be detected by the assay device (1). The antibodies may be raised against the enzyme *per se* or against a component thereof (e.g. a fragment thereof and/or an epitope thereof).

Preferably the substrate for the captured enzyme may be a phosphatase substrate or a compound with a phosphate donor group. Preferably, the substrate is an acid phosphatase substrate and most preferably the phosphatase substrate is 2-phosph-L-ascorbic acid trisodium (AsAP).

Preferably the reactive species used in the method may be a tetrazolium compound. Most preferably the reactive species may be a nitroblue tetrazolium compound. The reactive species may be nitroblue tetrazolium chloride (NBT). The reactive species may alternatively be a halonitrotetrazolium compound. The reactive species may preferably be an iodonitrotetrazolium compound. Preferably the reactive species is iodonitrotetrazolium chloride (INT).

The insoluble product is preferably a precipitate, most preferably a visually detectable or observable precipitate, preferably a coloured precipitate.

The insoluble product is preferably detected or observed visually or electronically. Most preferably it is detectable due to a colour change.

### KITS

The current invention may be presented in the form of a kit. Such a kit will enable the operator to perform the assay, preferably outside of the laboratory. The kit may provide one or more or all of the reagents necessary to perform the assay. Such reagents may be in a dried or dehydrated form. Dried reagents may be reconstituted by the sample in a solvent, preferably an aqueous sample. Preferably all of the reagents are dried except the selective indication means (50) or some component of the selective indication means (50). Such a kit may be known as a "Semi-Dry Format". Preferably all of the reagents are dried except the reactive species. Most preferably all of the reagents are dried except for a tetrazolium solution of either INT or NBT. The operator adds the liquid to the dried reagents to perform the assay.

Preferably the assay device (1) is supplied in the form of a stick or most preferably a strip. Most preferably the dried reagents are bound to the strip or stick as it is provided. Kits of the current invention may also comprise one or more bags (e.g. plastic bags), pipettes, syringes, tubes or test tubes. Preferably the strip or stick is contained in a housing, for example a plastic, card or cardboard housing.

The assay device of the current invention may be provided in a kit as shown in embodiment 1 of Figure 1 or embodiment 2 of Figure 1.

A kit of the current invention may comprise or be capable of forming the assay device and all of the components of the assay device including:-
(a) a placement region (10) onto which the sample can be placed;
(b) a matrix (20) operably connected to said placement region (10) such that the sample when present (such as placed) on said placement region (10) can migrate along said matrix (20);
(c) at least one distinct capture location (30) on said matrix (20), wherein each distinct capture location (30) is distanced away from the placement region (10), and wherein the sample can migrate across said distinct capture location (30);
(d) capture means (40) being present at or defining each distinct capture location (30), wherein said capture means (40) are capable of binding to said enzyme such that at least a portion of said sample of said enzyme is retained at at least one distinct capture location (30); and
(e) selective indication means (50) or at least a component thereof, to provide selective indication of the presence of active enzyme bound to said capture means.

A kit of the current invention may comprise all or substantially all of the components of the assay device (a)-(d) and wherein the selective indication means (50) are provided which are then added to the device in use.

A kit of the current invention may comprise all of the components of the assay device (a)-(d) and at least a component of said selective indication means and wherein the kit further comprises the other component(s) of the selective indication means (50) which are then added to the device in use.

A kit of the current invention may comprise all of the components of the assay device (a)-(e).

A solvent such as water - or other suitable aqueous solution - may be included with or added to a kit or device of the current invention. The same or any of the kit components may be dispersed, dissolved or re-hydrated in a solvent such as water, purified water or de-ionised water.

A kit of the current invention may be supplied with a score card or reference card. The intensity of a colour change may be evaluated using a score card or reference card, preferably wherein the operator compared the intensity of the colour reaction on the matrix (20) to the score card. Semi-quantitative measurement may be performed using the score card, for example wherein the amount of active enzyme is above or below a certain amount or concentration, for example above or below 300 FTU/g, may be determined using the score card.

The score card or reference card may be made of paper, card or other physical materials. Alternatively the score card or reference card may be viewed electronically, for example on a telephone, smartphone, computer or other computational device. The score card may be compared to an electronic reference and/or the scorecard may be read electronically.

### EXAMPLES

The present invention will be described by way of example only in which reference is made to the following Figures.

Part 1 of the Examples section provides details on assay devices and methods of the present invention for determining the presence of active phytase in a sample.

Part 2 of the Examples section provides details on methodology that may be used for assay devices and methods of the present invention for determining the presence of active xylanase in a sample.

### Antibody Generation for the Assay Devices and Methods of the Present Invention

Antibodies for use in the assay device and methods of the present invention can be generated by using the methodology of Harboe N, Ingild A. Immunization, isolation of immunoglobulins, Estimation of antibody titre. Scand J Immunol Suppl. 1973, 1:161-4).

In Part 1 of the Examples section, antibodies against the phytase BP17, for use in the assay device and methods of the present invention, were generated by following the methodology of Harboe N (1973) (*ibid*). The sequence for BP17 (excluding signal peptide) is shown above.

### PART 1

In the work leading up to and resulting in the present invention two different lines of investigation were taken.

One approach resulted in a device according to the present invention (generically represented in Figure 1) and methods using same and kits comprising same. This line of approach we called Format 1. This device etc. is discussed in detail in Examples 1 etc.

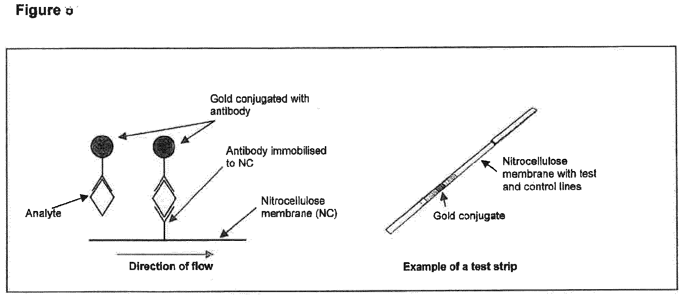

We also worked on a gold conjugate antibody sandwich lateral flow assay. This line of approach we called Format 2. For the reasons explained herein, this line of approach was deemed not to be suitable. Information regarding Format 2 (i.e. the gold conjugate antibody sandwich lateral flow assay), such as Figure 5 and its associated commentary, have been added for comparative reasons (see Comparative Example Section).

In addition, in the Comparative Example Section we performed a direct comparison of Format 1 with Format 2 for one aspect of the experiments.

### EXAMPLES 1 - 9

### Format 1 - Assay Devices and Methods of the Present Invention

### Example 1

The assay and device of the present invention relates to an antibody capture with precipitating substrate test (e.g. Figure 1 and Figure 2 and Figure 3).

In this respect, the antibody capture with precipitating substrate test was characterised. A suitable acid-phosphatase substrate was identified, with the main feature being the reduction of a tetrazolium salt to produce a coloured precipitate, and subsequently shown to function in a lateral flow-type assay with a dose-dependent signal response.

Several hurdles in the development of an antibody capture with precipitating substrate test were noted, such as the substrate complexity and the generated precipitate migrating due to test flow. However, this test was more suitable than the gold conjugate sandwich assay (Figure 5) as it directly measures enzyme activity rather than simply relying on the binding of the enzyme to antibody.

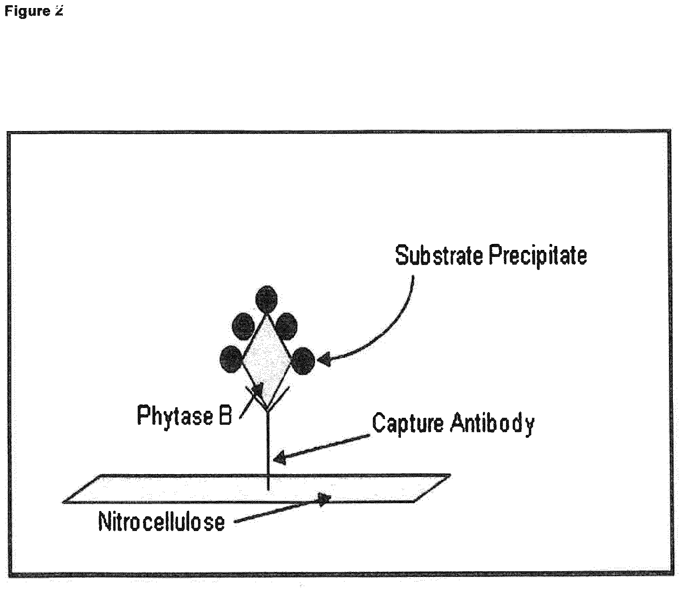

The antibody capture with precipitating substrate assay (Figure 2) was extensively developed. Multiple formats were investigated and the test refined from an initially complex design with a multi-component substrate and post sample addition test stick manipulation to a simple configuration with minimal user steps.

The semi-quantitative aspect of the test is determined by a three line test region where a three line result represents high levels of active enzyme (a pass result), two lines represents intermediate levels (borderline) and one or zero lines indicating low enzymes levels (fail).

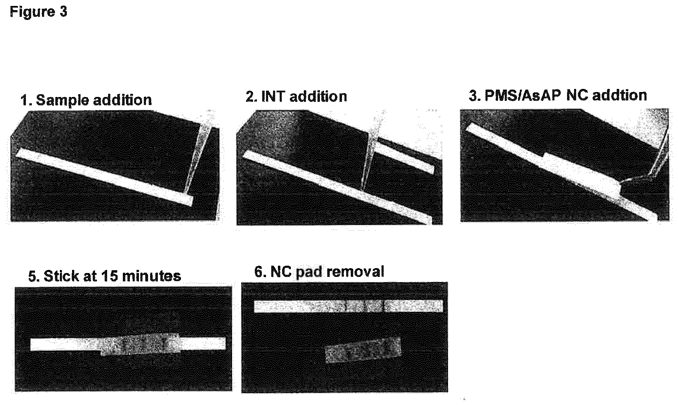

The evolution of this assay resulted in the development of a very useful format - namely what we call the "Semi-Dry Format". Figure 3 shows the steps involved in the Semi-Dry Format of the present invention.

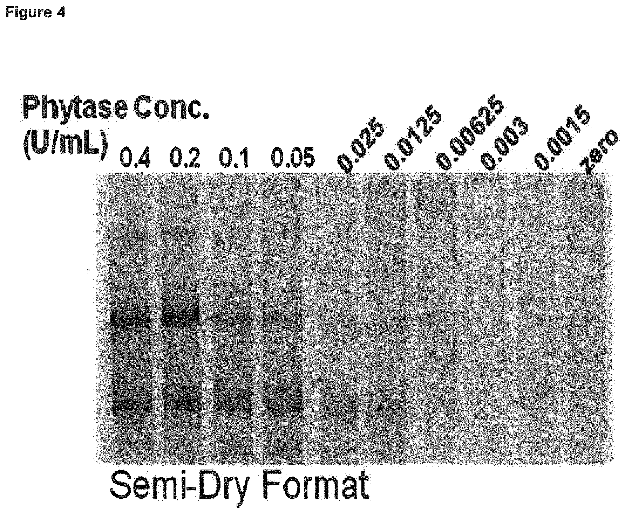

In this respect, and as illustrated in Figure 3, the Semi-Dry Format requires the operator to produce a test sample in water which is applied to the test stick. One component of the substrate complex in solution is applied 'straight from the bottle' (no preparation required by the operator) directly to the test stick matrix (nitrocellulose in this instance) which is covered with a pad containing the other substrate components which are dry. The test is then allowed to develop and the result read after 20-30 minutes (Figure 4). Here, Figure 4 presents results from a 6-phytase (called BP17) dilution series using the Semi-Dry Format of Figure 3. The 6-phytase is derived from *Buttiauxella* sp. as described above.

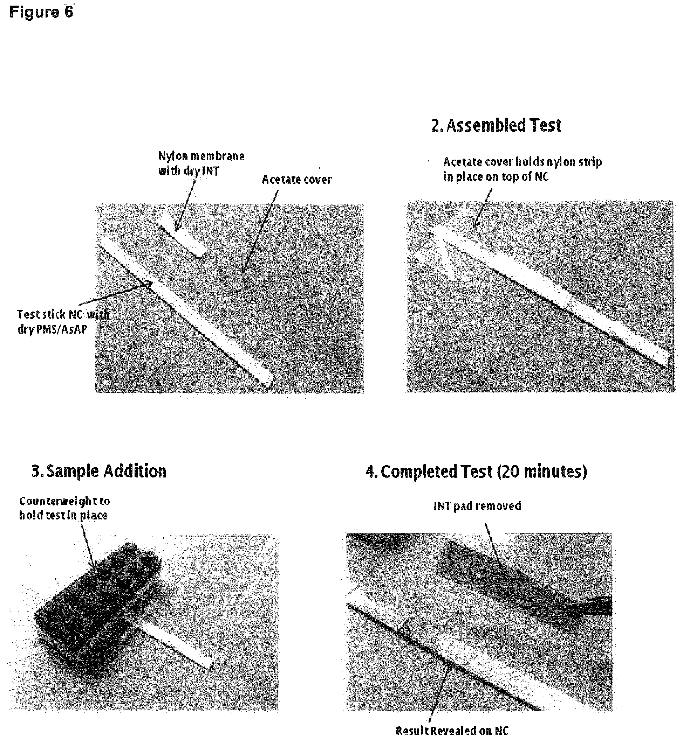

A "Dry Format" was also investigated where all the components are dried into the test so that the operator would have to only apply sample (Figure 6). Here the operator simply adds the sample and reveals the test. A final test could be cassetted and the counterweight depicted in Figure 6 would not be required. In the example illustrated, a phosphate donor 2-phospho-L-ascorbic acid trisodium (AsAP) and reaction enhancer phenazine methosulphate, (PMS) are dried into the test nitrocellulose matrix (NC) and a nylon pad of tetrazolium substrate, iodonitrotetrazolium chloride (INT) is placed securely on top. The liquid sample is added as usual, and it is the sample solution itself which rehydrates the substrate components.

Thus, in Figure 6 (namely an example of a Dry Format test) the operator simply adds the sample and reveals the test. In this Figure, 2-phospho-L-ascorbic acid trisodium = **AsAP;** reaction enhancer phenazine methosulphate = **PMS;** Nitrocellulose matrix =NC; and tetrazolium substrate iodonitrotetrazolium chloride = INT.

### Example 2 - Sample Preparation

### Solvent

The method of sample preparation was performed in water. For the purposes of continuity MilliQ water has been used. MilliQ refers to water that has been purified and deionised to a high degree by a water purification system.

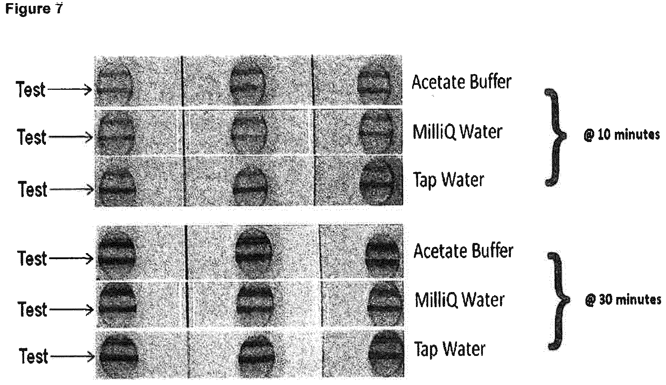

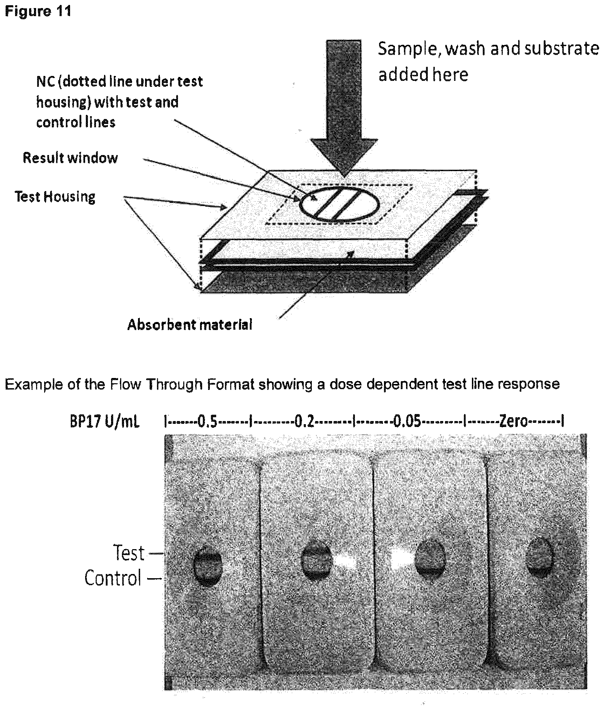

Figure 7 shows the results obtained when 1 gram of feed pellets were extracted for 30 minutes in either 0.25 M acetate buffer, pH 5.0, MilliQ water or tap water and run on the flow through format in triplicate (see also Figure 11). In each case the bottom line represents the test line and the top line is the control line. The tests were photographed at 10 and 30 minutes after substrate application.

Experiments have shown that use of tap water also produces satisfactory results with the semi-dry format. Tap water gives a slightly increased visual signal compared to the other two solvents tested.

### Agitation Time

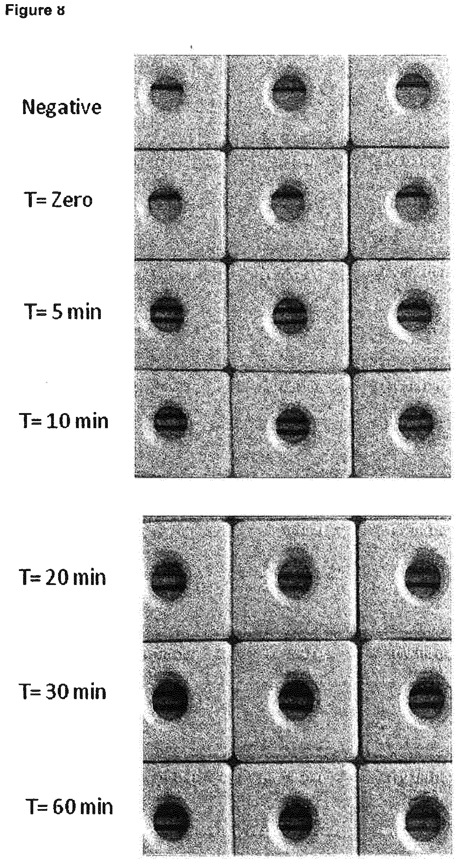

In the example illustrated in Figure 7, 1 gram of feed pellets were extracted in 10 mLs of MilliQ water and the extraction allowed to proceed for 10 minutes with constant agitation. However, further experiments have shown that simple shaking of the extraction for 5 minutes produces a full extraction of the phytase BP17 into the aqueous phase (Figure 8). Thus the preparation of the test sample is a quick and simple procedure, manageable by an operator with minimal training.

Figure 8 shows the results of sampling at different time intervals. 1 gram of feed pellets were added to 10 mLs of MilliQ water and the resulting solution sampled at various time intervals from zero to 60 minutes. After 5 minutes the extraction appears to be sufficient for testing.

In one aspect, the operator will fill a sample extraction tube or bag to a predetermined level (marked by a line on the tube) with feed pellets and then fill the tube to a second line with tap water. The lines will be marked so as to optimise the feed to water ratio. Although a ratio of 1:10 has been used throughout, this ratio could be altered to vary the effective enzyme concentration if needed.

In the experiments presented here a blank feed mash (lacking phytase BP17) was used and extracted as above and purified phytase BP17 added to this to give defined amounts of enzyme.

### Example 3

### Antibody Capture and Precipitating Substrate Test Development

### The Precipitating Substrate

For the activity assay a substrate was identified. This substrate is a generic acid phosphatase substrate using a phosphate donor (2-phospho-L-ascorbic acid trisodium, **AsAP**) a reaction enhancer (phenazine methosulphate, **PMS**) and a reactive species (nitroblue tetrazolium chloride, **NBT**). In the presence of these chemicals and acid phosphatase the soluble tetrazolium salt will be reduced resulting in the production of an insoluble precipitate (the formazan derivative of the tetrazolium salt). In the case of nitroblue tetrazolium this results in the formation of a blue/purple precipitate.

### Test Format Development

Several formats have been analyzed during the feasibility phase of the project.

### Flow Through Format.

This approach utilises a 'flow-through' methodology. In contrast to standard lateral flow methodology a flow through test involves the application of sample directly on top of a specialized nitrocellulose (striped with anti-phytase BP17 antibodies) and the sample is drawn through the nitrocellulose by way of an underlying absorbent pad. After sample application the substrate is applied in the same way and the test is then allowed to develop (Figure 11).

Figure 11 is a schematic representation of the Flow Through Format. The format of Figure 11 is very simple and gives a clean visual result. However, for some aspects, it may be desirable to have an even better semi-quantitative format. Thus, to facilitate the operator in the interpretation of the results in this manner it was decided to produce a multiple line test formats where the number of test lines visible after test completion would indicate the levels of enzyme present in the sample.

### Dip Stick Format.

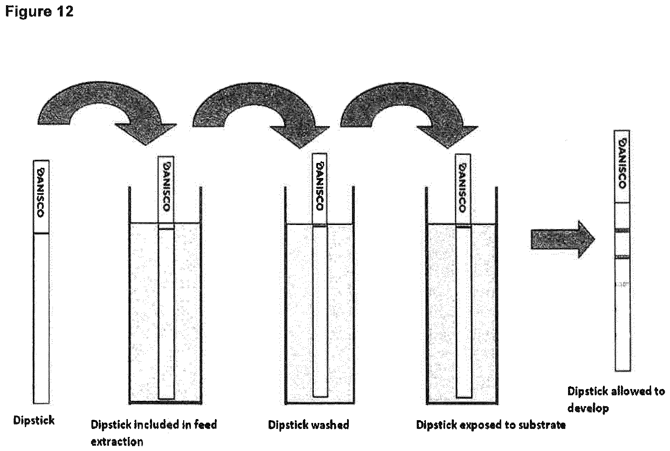

This format adopts the multiple line approach. Initially the nitrocellulose was striped with 6 lines of increasing concentrations, and the whole test stick was submerged (dipped) in the feed extract sample. After incubation the stick is rinsed in water and then the substrate solution is applied and the test is allowed to develop (Figure 12).

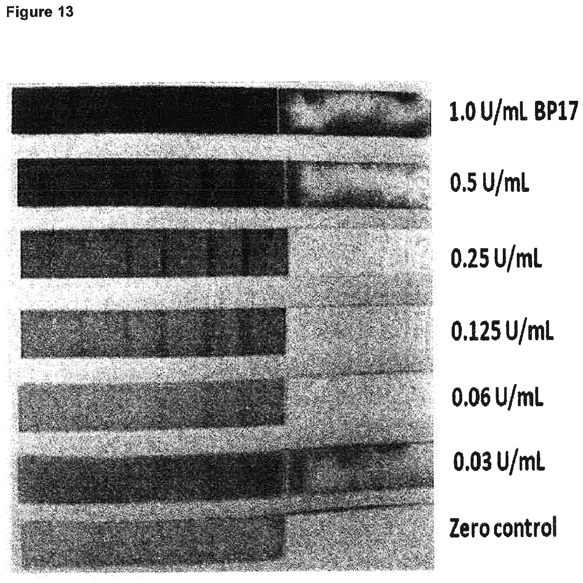

Figure 12 shows a schematic representation of the Dip Stick Format. This format was very easy to perform and one advantage was that there were no absorbent pads present on the test stick, which meant that no post sample test stick manipulations were required. The test functioned but there are instances when an even better semi-quantitative result is desired. Here, all of the test lines were visible when the enzyme tested - phytase BP17 - was present, albeit at dose-dependent visual intensities. Ideally test lines with lower anti-phytase BP17 concentrations would have not appeared when challenged with low level phytase BP17 samples but this was not the case (Figure 13).

Figure 13 shows the Dip-stick format test results. Here, nitrocellulose was striped with 6 test lines at 4, 2, 1, 0.5, 0.25 and 0.125 mg/mL anti-phytase BP17 (right to left on photograph). Test sticks were added directly to the feed extraction tubes (7 mL, with 0-1.0 U/mL phytase BP17) and incubated for 20 minutes with shaking. Sticks were then removed, briefly rinsed in MilliQ water and substrate added to the sticks. Tests were allowed to develop for 30 minutes and then photographed. The result does not appear to be semi-quantitative and it appears that a high background was also seen, which was proportional to the amount of phytase BP17 in the sample.

### Wicking Stick Format.

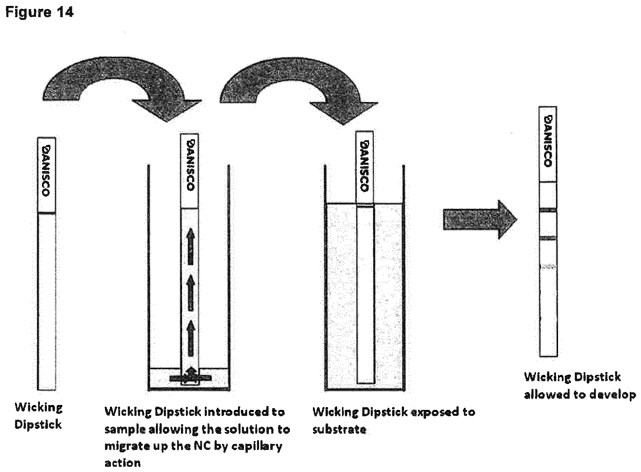

Here a similar test stick to the Dip Stick was used, but with the test run in a different manner. Rather than submerging the whole test stick, here the sample was allowed to migrate along the test stick by capillary action. Once complete, the substrate was applied in a similar fashion and test line development allowed to proceed (Figure 14).

Figure 14 presents a schematic representation of the Wicking Stick Format. This test format was run vertically with the sample in a microtitre plate well and once the sample had been run the stick was removed from the well and placed horizontally to allow substrate addition.

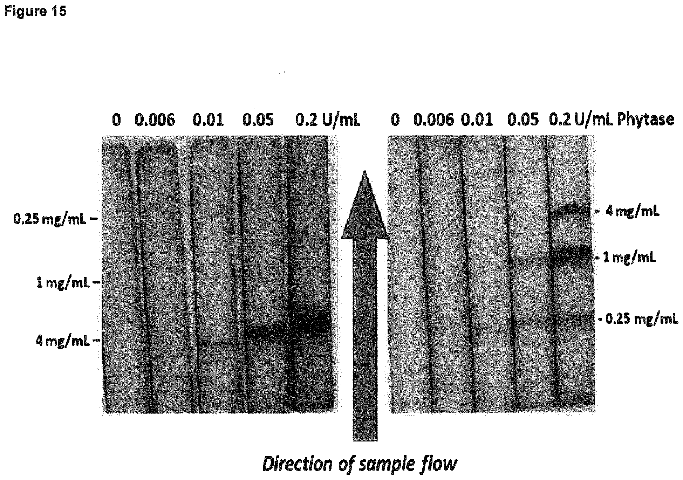

Initially a multiple line test with varying amounts of anti-phytase BP17 was employed and it was shown that the order in which the sample encountered the lines was important for test development. It appeared that phytase BP17 in the sample would bind the first test line, and if there was still unbound phytase BP17 this would proceed to the second line and so on. Thus if the first test line contained a high level of anti-phytase BP17 antibodies then the majority of the sample could bind to that line leaving little remaining to bind subsequent lines. On the other hand if the first line encountered contained low levels of antibodies then this line could become quickly saturated and the majority of the phytase BP17 would continue on to the next test line (Figure 15). This shows that the lowest level of antibodies should preferably be closest to the placement region.

Figure 15 shows the Wicking stick format test results. Here, nitrocellulose was striped with test lines at 4, 1 and 0.25 mg/mL anti-phytase BP17. Samples were diluted in water at 0.2, 0.05, 0.012, 0.006 U/mL phytase BP17. Samples wicked up the NC by touching the end to the solution and allowing the solution to travel up the stick by capillary action. Two orientations of stick tested: From 4 to 0.25 mg/mL, and from 0.25 to 4 mg/mL. After 10 minutes sticks were exposed to substrate solution and allowed to develop for 30 minutes. The orientation of the test line concentrations has a significant impact on the successful running of the test.

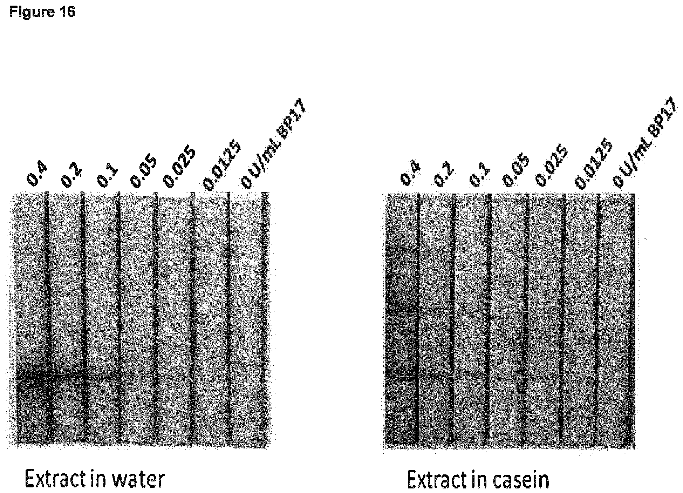

To simplify the test system experiments with multiple lines at single concentration of anti-phytase BP17 were tested. Initially it appeared that the vast majority of the phytase BP17 in the sample was binding the first test line. However this perceived problem was circumvented by the addition of blocking reagents to the sample, with the milk protein casein showing the best results (Figure 16).

Figure 16 shows the Wicking stick format test results using different blocking agents. Samples were prepared at 0.4, 0.2, 0.1, 0.05, 0.025 U/mL phytase BP17 and zero controls in blank mash extracts (either water or casein). Test sticks were placed in wells of microtitre plates containing 40 µL samples and sample allowed to wick up the stick for 10 minutes. Sticks were blotted dry, laid flat and 40 µL of substrate solution pipetted onto the nitrocellulose. Sticks were allowed to develop for 30 minutes and then photographed (see Example 6).

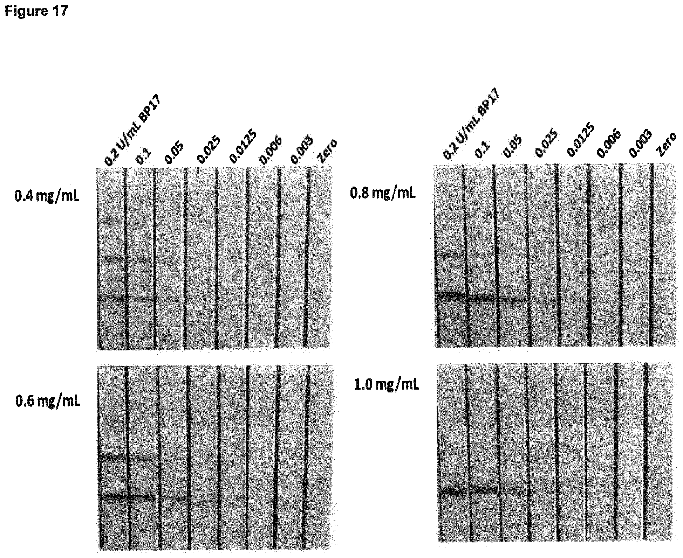

Figure 17 shows results relating to varying the test line concentration to modulate the semi-quantitative nature of the test (Wicking stick format). The results show that the concentration of the test line antibody influences the semi-quantitative nature of the test with a concentration of 0.4-0.6 mg/mL appearing to be optimal for the required specification and thus shows that the sample is capable of transversing across the capture means.

In more detail, Figure 17 shows the results from varying the test line concentration to modulate the semi-quantitative nature of the test, using the wicking stick format. Nitrocellulose was striped with three test lines of the same concentration, either 0.4, 0.6, 0.8 or 1.0 mg/mL anti-phytase BP17. Blank mash extract was prepared with 1 g mash in 10 mL 50 mg/mL casein solution incubated for 10 minutes on the roller mixer. Pure phytase BP17 was added to mash extract at 0.2, 0.1, 0.05, 0.025, 0.0125, 0.00625 and 0.003125 U/mL. 40 µL samples pipetted into wells of microtitre plate, sticks inserted and sample allowed to migrate up the stick by capillary action for 10 minutes. Sticks were removed, blotted, and 40 µL substrate solution pipetted onto the sticks and allowed to develop for 30 minutes. The concentration of the test line antibody influences the semi-quantitative nature of the test with a concentration of 0.4-0.6 mg/mL appearing to be optimal for the required specification. These results show that, with adequate blocking, varying the test line concentration could be used to modulate the semi-quantitative nature of the test.

As with the Dip-Stick format, the advantage of the wicking stick format was that no absorbent pads were present in the test stick and thus the operator would not have to manipulate the test stick post-sample and pre-substrate additions. However a vertical format may not be highly desirable for some uses, especially if the stick would have to be re-orientated prior to substrate addition.

To address this, a horizontal format was developed with very similar materials, the only addition being an absorbent sample pad upstream of the nitrocellulose portion of the test stick. This approach gave a high quality result and opened up the possibility of further development which would not have been possible with the Format 2. In some instances, the addition of blockers may be required. In some instances, the sample pad may need to be removed prior to substrate addition.

### Example 4 - Tertrazolium substrates

In this example, a tetrazolium substrate was tested, iodonitrotetrazolium chloride (**INT**). This substrate resulted in a red precipitate on the test line (rather than blue/purple with NBT).

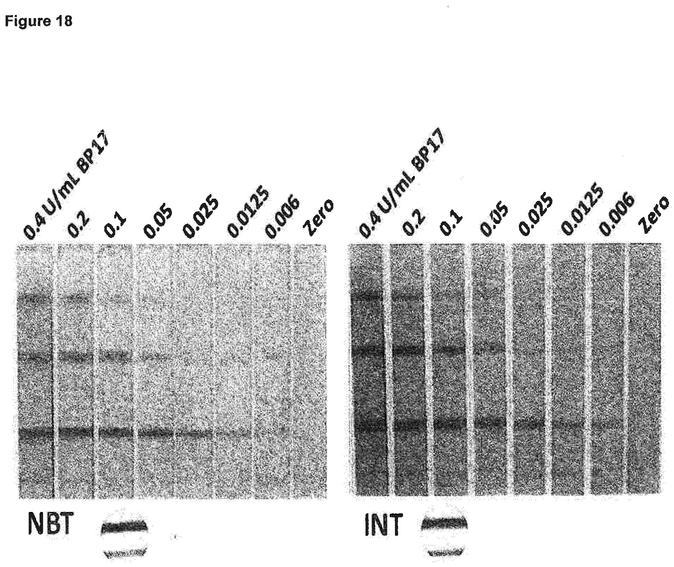

Figure 18 shows a comparison between NBT and INT with the Wicking Stick test. Blank mash extract was prepared with 1 g mash in 10 mL 50 mg/mL casein solution incubated for 10 minutes on the roller mixer. Pure phytase BP17 was added to mash extract at 0.4, 0.2, 0.1, 0.05, 0.025, 0.0125 and 0.00625 U/mL. 40 µL samples pipetted into wells of microtitre plate, sticks inserted and sample allowed to migrate up the stick by capillary action for 10 minutes. INT solution was made at 4.45 mg/mL in acetate buffer, this gives the same molarity as the NBT solution. Sticks were removed, blotted, and 40 µL substrate solution pipetted onto the sticks, incubated for 1 minute. The substrate solution was then removed, sticks blotted and allowed to develop for 30 minutes.

As can be seen from Figure 18, the results showed that using INT as a substrate gave similar results to NBT. Both NBT and INT were used interchangeably from this point onwards.

### Example 5 - Nitrocellulose types

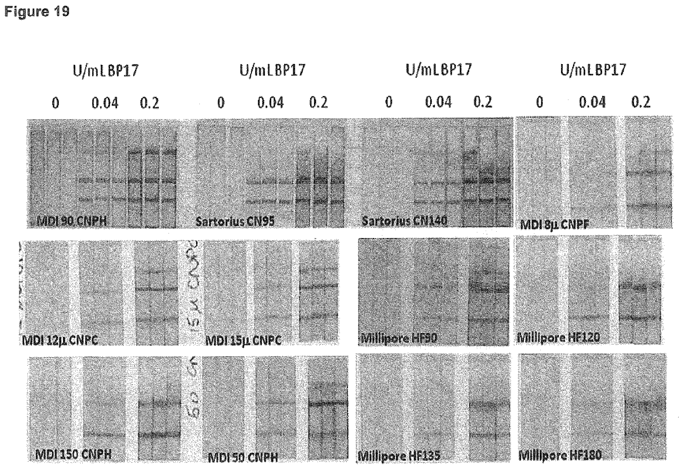

As the horizontal format was regarded as a promising format an experiment was performed testing a wide range of nitrocellulose types in order to determine the most appropriate for this format (Figure 19).

Figure 19 presents the results of testing a wide range of nitrocellulose types in order to determine the most appropriate. A variety of nitrocellulose types were striped with three test lines at 0.5 mg/mL anti-phytase BP17. Phytase BP17 samples were diluted from purified enzyme in blank feed mash extract (1 g in 10 mLs water) to give 0.2 and 0.04 U/mL phytase BP17 (plus feed only zero control). Extracts were pipetted onto the sample pads of tests sticks and sample was allowed to migrate along the stick by capillary action to completion. Sample pads were removed and 40 µL of substrate applied. The test was allowed to proceed to 20 minutes and then photographed.

One of the nitrocellulose membranes tested, namely MDI 90 CNPH showed the best results (see Figure 19) and indeed had been the nitrocellulose of choice as determined from previous experiments with previous formats. This nitrocellulose is readily available. Two other nitrocellulose types (MDI SS12 12µ and SS12 15µ) also gave satisfactory results. All membranes tested gave a response.

### Example 6 - Blocking reagents

Up to this point the blocking reagent (e.g. casein) had been added to the sample, post extraction. It was decided that this was not ideal as it would require the solution to be supplied with the final test and would require the operator to add it to the feed extract. Experiments were performed to address this. Casein can be difficult to dissolve in water. However a sodium salt of casein was found to be better in this respect. This allowed the possibility of adding sodium casein in defined amounts directly to the feed extraction. This approach was functional. However, for some aspects, this approach could be even further optimised since it would require defined amounts of sodium casein to be supplied in the sample extraction tube which could be displaced during shipping and lost upon opening of the tube. Hence, it was decided to test the possibility of supplying the blocking reagent within the test stick itself.

Experiments were designed to place the blocking reagent dried into a pad between the sample pad and the test nitrocellulose.

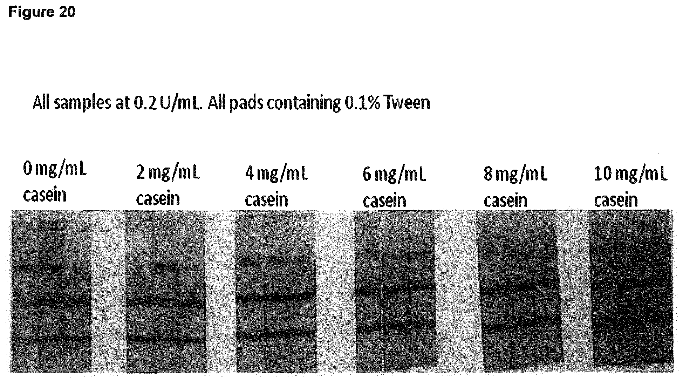

Figure 20 shows the results of testing reducing the concentration of blocking agent casein in the presence of surfactant (Tween-20). Polyester conjugate pads were saturated with casein solutions at various concentrations and dried at 37°C. Pads were then placed 'upstream' of the test sticks (together with an additional sample pad). Blank mash was extracted in water (1 g in 10 mL) and purified enzyme diluted in the extracts. Samples were pipetted onto the sample pad which then flowed through the casein containing pads and onto the test sticks. Sample and conjugate pads were removed and 40 µL of substrate applied. The tests were allowed to proceed to 20 minutes and then photographed. A surprising result was seen, in that the presence of the surfactant alone was sufficient to allow the test to perform satisfactorily. Thus, the pad with no casein showed the same colour intensity as the pads with casein.

Encouraged by this finding, experiments were designed to add the blocking reagents directly onto the test nitrocellulose in order to determine whether this approach would also give a satisfactory result.

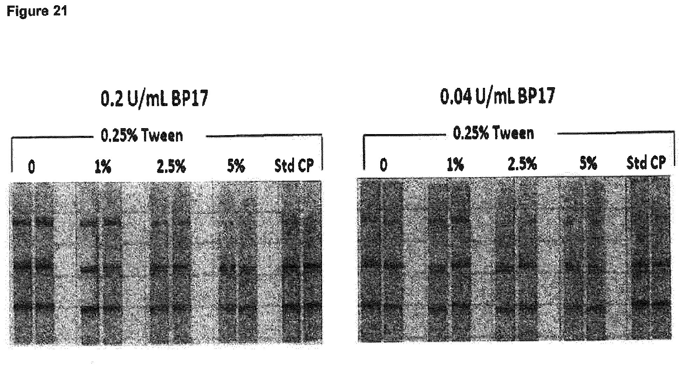

Figure 21 shows the results obtained by adding the blocking reagents directly onto the test nitrocellulose to test if satisfactory results could be obtained.

In more detail, Figure 21 illustrates the results of an experiment to add the blocking reagents directly to the nitrocellulose. Lengths of nitrocellulose were incubated in various casein/Tween solutions for 5 minutes and then dried at 55°C for 10 minutes. Nitrocellulose was laminated to backing card with untreated sample pad (and no conjugate pad). A set of control sticks with unblocked NC and casein/Tween conjugate pad were also constructed. Phytase BP17 samples were diluted from purified enzyme in blank feed mash extract (1 g in 10 mLs water) to give 0.2 and 0.04 U/mL phytase BP17 (plus feed only zero control). Extracts were pipetted onto the sample pads of tests sticks and sample was allowed to migrate along the stick by capillary action to completion. Sample and conjugate pads were removed and 40 µL of substrate applied. The tests allowed to proceed to 20 minutes and then photographed.

Again a situation where the surfactant alone in the absence of casein gave excellent results (Figure 21).

A dilution series of phytase BP17 concentrations was tested with surfactant-only blocked NC with both INT and NBT substrates to test the sensitivity of this method.

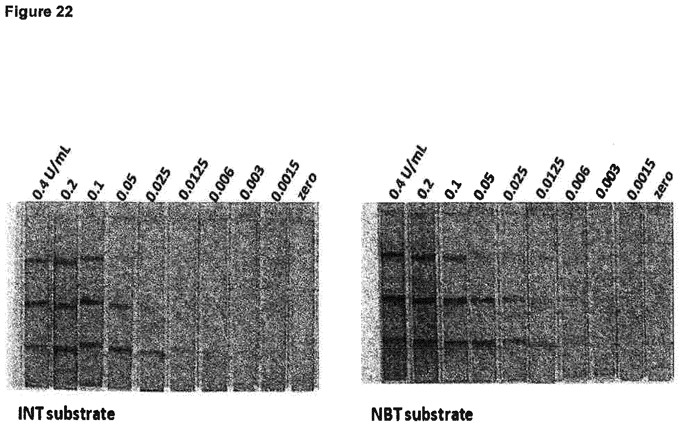

Figure 22 shows the results of a dilution series of phytase BP17 concentrations tested with surfactant-only blocked NC with both INT and NBT substrates to test the sensitivity of this method. Striped nitrocellulose was incubated in 0.25% Tween-20 solutions for 5 minutes and then dried at 55°C for 10 minutes. Phytase BP17 samples were diluted from purified enzyme in blank feed mash extract (1 g in 10 mLs water) to give a dilution series from 0.4-0.0015 U/mL phytase BP17 (plus feed only zero control).

In more detail, Figure 22 shows the results of this experiment. Striped nitrocellulose was incubated in 0.25% Tween-20 solutions for 5 minutes and then dried at 55°C for 10 minutes. Phytase BP17 samples were diluted from purified enzyme in blank feed mash extract (1 g in 10 mLs water) to give a dilution series from 0.4-0.0015 U/mL phytase BP17 (plus feed only zero control). Extracts were pipetted onto the sample pads of tests sticks and sample was allowed to migrate along the stick by capillary action to completion. The sample and conjugate pads were removed and 40 µL of substrate (NBT or INT based) applied. The tests were allowed to proceed to 20 minutes and then photographed. The results show that both substrates performed similarly and the overall sensitivity and semi-quantitative nature of the method appeared excellent.

The appeal of a method of directly blocking nitrocellulose with surfactant is that it removes the necessity of including a secondary 'blocking pad' on the test stick and thus eliminates a level of complexity with respect to test assembly. Moreover inclusion of the blocking reagent in the test itself negates the requirement of the operator to do anything other than add feed and water to an empty tube for the sample preparation.

The test stick produced is a very simple design where it comprised of test nitrocellulose striped with three lines of anti-phytase BP17 antibodies, all at the same concentration and blocked, post striping, with 0.1% Tween-20 solution in water attached to a blank untreated sample pad.

### Example 7 - Substrate Delivery Experiments

In all the experiments detailed above the substrate solution was added by mixing the three components together (AsAP, PMS and tetrazolium, INT or NBT) immediately prior to use. In order to simplify this for the operator, experiments were performed to attempt to dry these components into the final test format. This would mean the operator would not be responsible for substrate composition and would negate the requirement of supplying liquid test components.

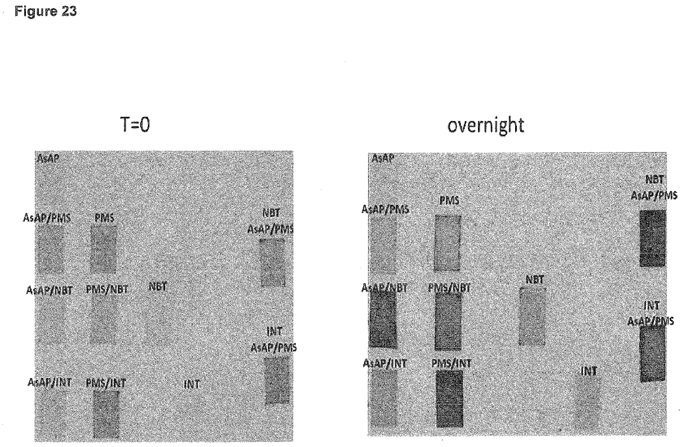

Initial experiments were performed where the various substrate components were mixed together and used to saturate absorbent pads followed by drying and storage at 37°C and the results are shown in Figure 23.

Figure 23 shows the results obtained when testing drying and storing the various substrate components mixed together. 1 cm squares of absorbent were soaked in various combinations of the (premixed) substrates for 5 minutes. Pads were blotted dry and placed in an incubator at 37°C. At 30 minutes the pads were deemed to be dry and this was regarded as 'Time=0'. Pads were monitored at regular time intervals, specifically looking for purple-biased (NBT) or red-biased (INT) colour changes. Time zero and overnight are shown.

In this experiment, 1 cm squares of absorbent were soaked in various combinations of the (premixed) substrates for 5 minutes. Pads were blotted dry and placed in an incubator at 37°C. At 30 minutes the pads were deemed to be dry and this was regarded as 'Time=0'. The pads were monitored at regular time intervals, specifically looking for purple-biased (NBT) or red-biased (INT) colour changes. Time zero and overnight are shown in Figure 23. After overnight it appears the more useable combinations are: NBT or INT on its own; AsAP + PMS; PMS; AsAP + INT appears moderately stable, and more stable than AsAP + NBT.

From this experiment it was clear that mixing the tetrazolium solution, both INT or NBT with any of the other components of the test resulted in eventual precipitation of the tetrazolium over time.

It was also demonstrated that mixing and drying of the AsAP and PMS appeared stable over time. Experiments to utilise all these dry components have been performed, as have experiments where some of the substrate components are dry while others are used in solution. It appears possible that all of the components of the assay could be provided in dried form. Potentially use of an aqueous sample could rehydrate the dried components.

Thus, in Figure 6 (an example of a Dry Format test) the user (operator) simply adds the sample and reveals the test. In this Figure, 2-phospho-L-ascorbic acid trisodium = AsAP; reaction enhancer phenazine methosulphate = PMS; Nitrocellulose matrix = NC; and tetrazolium substrate iodonitrotetrazolium chloride = INT

### Example 8 - The Dry Format

Experiments were performed to dry all the test components into the test so that the operator simply has to add an extract to the test and then read the result, without any further additions or manipulations. Here pads, with all the components dried down, would be incorporated into various parts of the test system. As was shown previously all three components cannot be premixed and dried into a single pad, and so a test a system where the various components can be separated, or at least utilizing combinations which are stable, should be determined as also their various locations within the test. An example of how such a test is envisaged can be seen in Figure 6. Thus, Figure 6 presents a diagram of a Dry Format test.

In the Dry Format test of Figure 6 the operator simply adds the sample and reveals the test. A final test could be cassetted and the unique counterweight depicted in Figure 6 would not be required. In the example illustrated PMS/AsAP is dried into the test nitrocellulose and a nylon pad of INT is placed securely on top. The sample is added as usual, and it is the sample solution itself which rehydrates the substrate components

### Example 9 - The Semi-Dry Format

As an alternative to the Dry Format (above), experiments where some of the components were supplied in a dry form and others remain in solution have been performed. This has produced a test system referred to as the Semi-Dry format.

In the Semi-Dry Format the sample is run along the horizontal stick as before, and once complete the tetrazolium solution is pipetted directly onto the nitrocellulose of the test. This is then covered with a nitrocellulose pad containing dried AsAP/PMS and the test allowed to develop. This is illustrated in Figure 3 (Steps involved in the Semi-Dry Format).

This format has several advantages. The operator does not have to prepare the substrate reagents prior to the test, and although the format requires the tetrazolium solution to be provided it is simply a case of adding it 'straight from the bottle'. One advantage observed with this format is that the requirement of the absorbent pad removal is eliminated. It appears that the presence of the AsAP/PMS pad fixes the precipitate in place and even in the presence of the sample pad the resulting precipitate does not migrate.

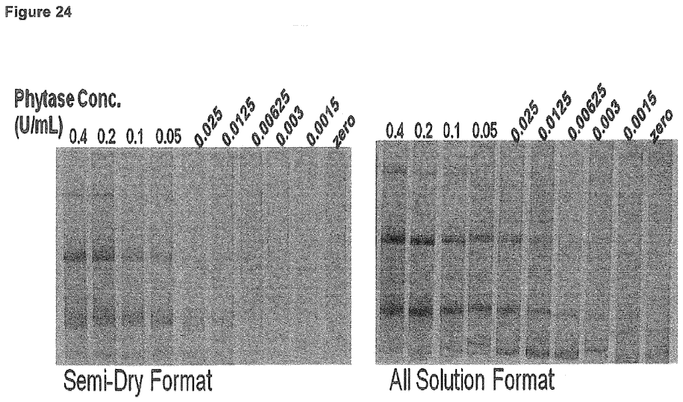

A comparison between the Semi-Dry format and the previous (all solution) format is shown in Figure 24.

Figure 24 shows a comparison between the Semi-Dry Format and the all solution format. Phytase BP17 samples were diluted from purified enzyme in blank feed mash extract (1 g in 10 mLs water) to give a dilution series from 0.4-0.0015 U/mL phytase BP17 (plus feed only zero control). Extracts were pipetted onto the sample pads of tests sticks and sample was allowed to migrate along the stick by capillary action to completion. For the All Solution format the sample pads were removed and 40 µL of complete substrate applied. For the Semi-Dry format 20 µL of INT solution was added to the test nitrocellulose and a pad of PMS/AsAP placed on top. The tests were allowed to proceed to 20 minutes and then photographed.

The results of the experiment of Figure 24 show that sensitivity of the Semi-Dry method is acceptable with regard to the test specifications.

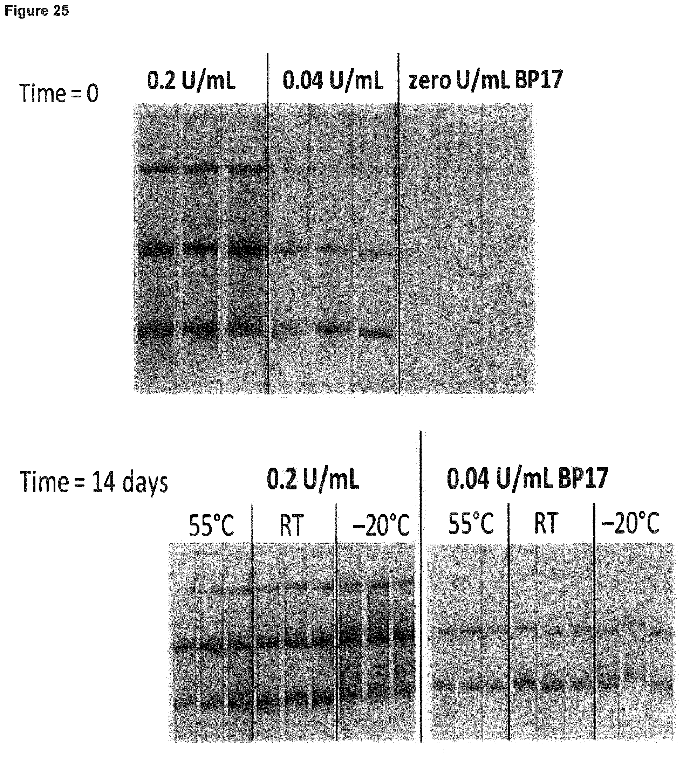

In order for this semi-dry format to be acceptable it is important that the tetrazolium solution remain stable for the proposed shelf-life of the final test. Experiments have been initiated to address this. The results of such an experiment are shown in Figure 25.

In the experiment of Figure 25, phytase BP17 samples were diluted from purified enzyme in blank feed mash extract (1 g in 10 mLs water) to give 0.2 and 0.04 U/mL BP17. INT was prepared at 40 mg/mL in 50% methanol, 0.125M acetate buffer heated at 60°C to dissolve. This stock solution was diluted to 10 mg/mL in 0.25M acetate buffer. Aliquots were stored frozen at -20°C, at room temperature and at 55°C. 40 µL of each extracts was pipetted onto the sample pads of tests sticks and sample was allowed to migrate along the stick by capillary action to completion. Tests were performed in triplicate. 20 µL of INT solution was pipetted onto the test NC and a strip of PMS/AsAP NC placed on top. Test allowed to proceed to 20 minutes and photographed. A 'time = zero' test was performed immediately with the freshly prepared INT solution to give a baseline signal. After 14 days the frozen, RT and 55°C were visually compared to give an ongoing comparison of the stability of the INT solutions. After 14 days no deterioration of the INT solution was seen RT or 55°C. With the -20°C tests the signal was less than the other temperatures which is believed to be due to some INT precipitation during storage and thawing.

As after 2 weeks at all temperatures no difference in activity was noted, this suggests that the tetrazolium solution has good stability over time (Figure 25).

### Example 10 - kit

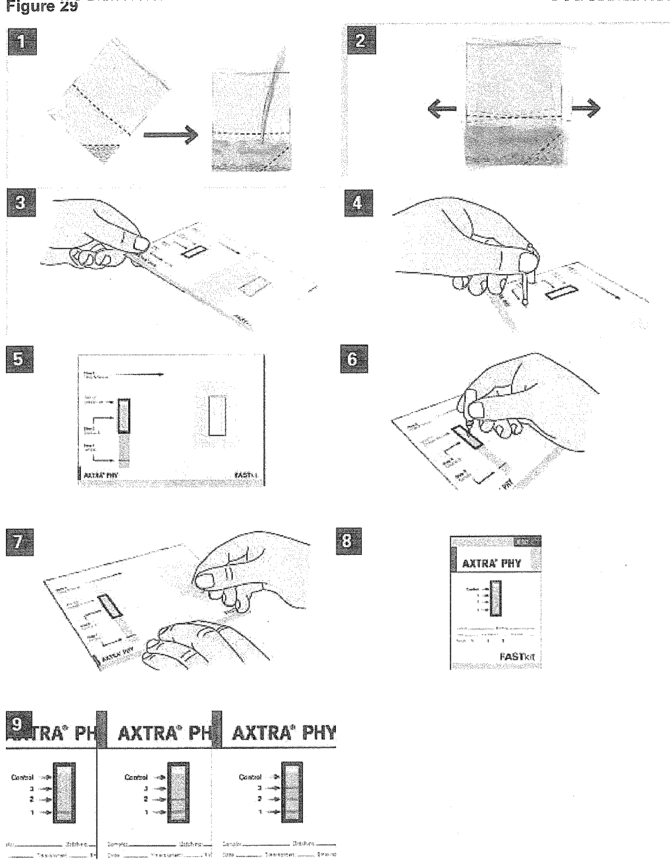

Figure 29 illustrates the use of a device/kit/assay of the invention.

In panel 1 of Figure 29 a collection bag is filled with sample to the first line indicated, then the bag is filled with water to the second line indicated.

In panel 2 the bag is sealed and them shaken until the sample is broken down.

In panel 3 the housing (as per embodiment 1, illustrated in Figure 1) is opened like a book.

In panel 4 a pipette is filled with some of the sample extract from the bag, and this is applied to the placement region on the housing.

In panel 5 the user waits until the liquid has migrated to the top of the stick. This may be indicated by a colour change.

In panel 6 one or more components of the selective indication means are added to the device.

In panel 7 the housing is closed and sealed.

In panel 8 the housing is laid of a flat surface to develop.

In panel 9, one, two and three line results are shown.

### COMPARATIVE EXAMPLE

### Format 2 - Gold conjugate antibody sandwich

The gold conjugate antibody sandwich illustrated in Figure 5 discriminated between feed samples which contained high and low level phytase BP17 activity. The signal strength generated in this test could be attenuated by the inclusion of free anti-phytase BP17 antibodies, the concentrations of which were shown to be capable of shifting the linear response of the test into the desired phytase BP17 activity range.

However, with this methodology, the employed polyclonal anti-phytase BP17 rabbit sera react with both native and denatured enzyme.

In addition, with the gold sandwich assay work there was a suspicion that the inactive sample was actually being depleted of enzyme and that the inactivation procedure had destroyed the enzyme rather than a simple inactivation.

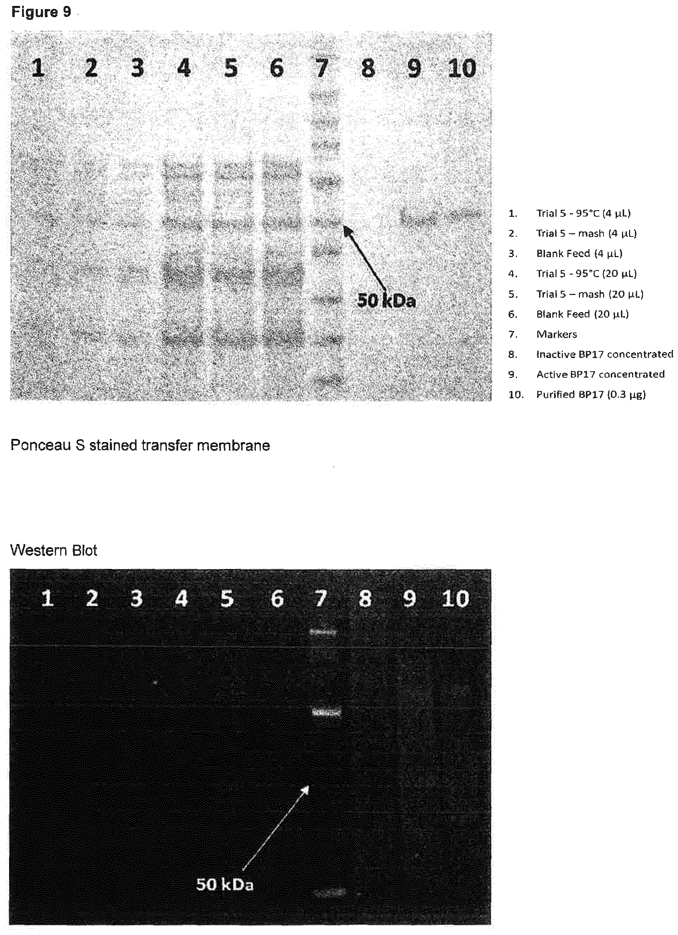

To address this, SDS-PAGE and Western blotting of the samples was performed and this showed that this theory was correct and that the inactive samples were indeed depleted of the enzyme (Figure 9).

Figure 9 relates to SDS-PAGE and Western blotting of feed samples and purified phytase BP17. Results showed that "Trial 5" pellets had very little detectable phytase BP17 compared to the mash, and that the inactive phytase BP17 had no discernable phytase BP17 band (although a general smear of material was seen on the Western blot). Purified phytase BP17 and active phytase BP17 (lines 9 and 10) gave one strong band in the protein stain but a multitude of bands in the Western Blot, probably due to background levels of phytase BP17 multimers and breakdown products, none of which was found with the heat-inactived sample (line 8).

In slightly more detail, Figure 9 shows the results of SDS-PAGE and Western blotting of feed samples and purified phytase BP17. Active and Inactive phytase BP17 samples were concentrated 30-fold using microconcentrators. The pellets and mash from "Trial 5" were used as this had been shown to have high level phytase BP17 in the mash and negligible amounts in the processed pellets. Feed pellets and mash were extracted in MilliQ water for 10 minutes and the resulting extractions run on SDS-PAGE. Proteins were transferred to a nitrocellulose membrane and stained for total protein with Ponceau S (left hand panel). Blot probed with anti-phytase BP17 antisera primary antibody, followed by biotinylated goat-anti-rabbit secondary antibody and visualised with streptavidin-Qdot 625 conjugate (Invitrogen). Results showed that Trial 5 pellets had very little detectable phytase BP17 compared to the mash, and that the inactive phytase BP17 had no discernable phytase BP17 band (although a general smear of material was seen on the Western blot). Purified phytase BP17 and active phytase BP17 gave one strong band in the protein stain but a multitude of bands in the Western Blot, probably due to background levels of phytase BP17 multimers and breakdown products, none of which was found with the inactive sample.

This finding cast into doubt the validity of the gold assay in determining the levels of active enzyme in a given sample. Although it seems likely that experimentally inactivated phytase BP17 and feed with low post-processing phytase BP17 activity would be mechanistically comparable (i.e. phytase BP17 being destroyed in both cases) the gold based assay would not be able to detect a situation where the phytase BP17 inactivation had occurred via denaturation rather than destruction.

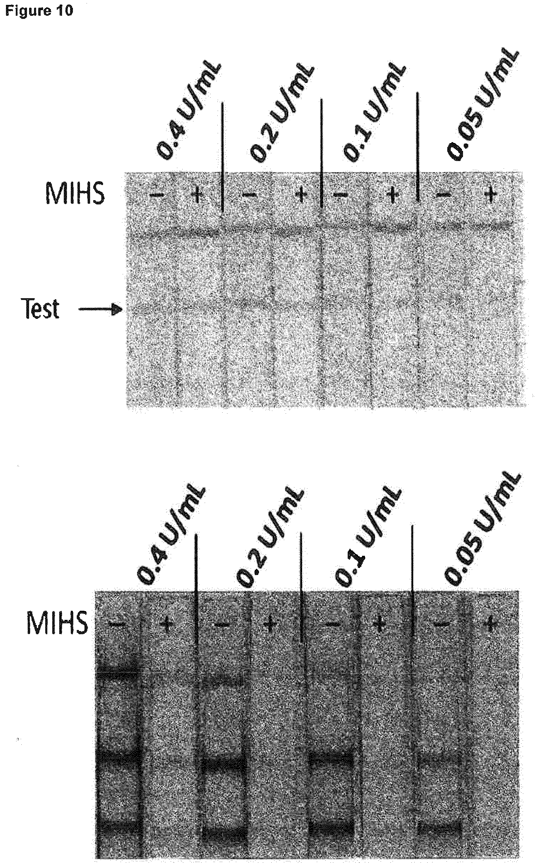

To test this the specific phytase BP17 inhibitor Myoinositol Hexasulphate (MIHS) was used to inhibit phytase BP17 activity without destroying the enzyme. Here the gold assay was still able to detect the presence of the inhibited enzyme whereas the activity assay showed a greatly decreased signal (Figure 10).

Figure 10 shows a comparison of the Gold Sandwich format (Format 2) and the Precipitating Substrate enzyme activity format (Format 1 - i.e. the assay of the present invention) using phytase BP17 specific inhibitor MIHS.

The results for the Gold Sandwich Test are presented in Figure 10 - at the top. Here, purified phytase BP17 was prepared in 75 mM Glycine-HCI buffer, pH 2.5 at various concentrations with or without 2.5 mM MIHS and allowed to incubate for 20 minutes before testing. Gold test were run against 100 µL samples.

By way of comparison, the Precipitating Substrate Tests are also presented in Figure 10 - at the bottom. The precipitating substrate tests were run against 40 µL samples at various concentrations. 40 µL of 2.5 mM MIHS in 75 mM Glycine buffer (or buffer only control) was applied to the test sticks and incubated for 15 minutes. The solution was then removed and substrate solution (AsAP, PMS, nitroblue tetrazolium chloride (NBT) in 2% Tween-20) added. This was incubated for 1 minute, removed and the sticks allowed to develop. The test was photographed after 20 minutes. The results suggest that the gold test is detecting the phytase BP17 whether it is inhibited or not whereas the precipitating substrate test is clearly detecting active enzyme only.

From Figure 10, which is a comparison of the Gold Sandwich format and the Precipitating Substrate enzyme activity format using phytase BP17 specific inhibitor MIHS (which inhibits phytase BP17 activity without destroying the enzyme) it can be seen that the gold assay (top) was still able to detect the presence of the inhibited enzyme whereas the activity assay (bottom) showed a greatly decreased signal.

Thus, the gold conjugate antibody sandwich format (Figure 5) only detects the presence of enzyme and not its activity. Functional inactivation of phytase BP17 with a specific inhibitor still gave a positive result with this form of the assay.

These results clearly demonstrated the need for an assay where the activity of an enzyme such as phytase BP17 could be measured directly, rather than relying on assumption that the enzyme was active simply because it was still present. The assay device of the present invention meets that need.

WO2005/014847 and WO2007/001895 describe an assay which is based on the same principle as the gold test. Allegedly, the assays only detect active phytase with measurement of heat-treated feed with the described Elisa kit. This may not be correct as the phytase is destroyed and removed together with the feed matrix in the sample preparation. The kit may therefore in reality not be able to discriminate between active phytase that is present and non-active phytase that is present.

### PART 2

In these experiments, we provide details on the detection methodology for detecting active xylanase in a sample using the device and methods of the present invention.

### Example i

The methodology used in this Example is a detection system based on a reducing sugar methodology. In this respect, we adapted the methodology described in Chong et al "Determination of reducing sugars in the nanomole range with tetrazolium blue" (Journal of Biochemical and Biophysical Methods, 11 (1985) 109-115). In that methodology (which is a plate assay), a reducing sugar (such as xylose) is detected by heating (95°C for 3 minutes or 55°C for 1 hour) the sugar sample with tetrazolium blue chloride and potassium sodium tartrate in NaOH.

In our methodology for detecting active xylanase in a sample using the device and methods of the present invention, we detect the production of the reducing sugar (e.g. xylose) by the enzymatic activity of xylanase on its xylan substrate by incubation at room temperature by substituting tetrazolium blue chloride with nitroblue tetrazolium chloride. Nitroblue tetrazolium chloride is from the same substrate family as is used in the phytase methodology described in Part 1 (above).

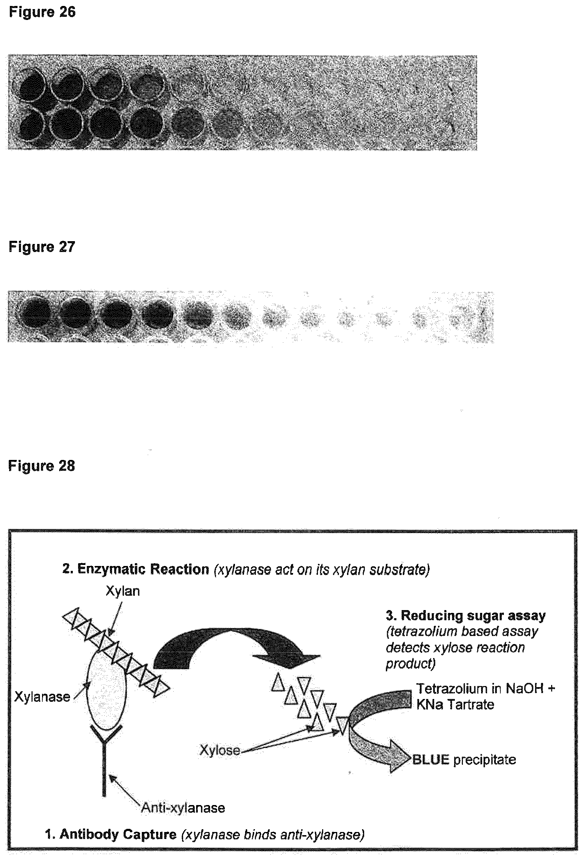

The data are presented in Figure 26. In this respect:
Top Row (of Figure 26), from left to right:
   Pure xylose (5 µL of 1% solution) followed by doubling dilutions (0.5-0.0001% xylose) and a zero control at the far right hand side
Bottom row (of Figure 26), from left to right:
   Xylanase assays containing 5 µL of 10 U/mL xylanase in the first well, followed by doubling dilutions of the enzyme (5-0.001 U/mL xylanase) and a zero control at the far right hand side. Each well also contained 5 µL of 1% xylan (substrate) in acetate buffer (0.25 M, pH 4.5). After xylanase and xylan were mixed together in each well the plate was incubated for 60 minutes at 37°C to allow the enzyme to react with its substrate.

In our methodology, 200 µL of 'developing solution' was added to each well and the plate placed at 55°C for 1 hour and then photographed. The developing solution contains 1 mg/mL Tetrazolium Blue, 0.5 M potassium sodium tartrate and 0.05 M NaOH.

Our reducing sugar methodology detects 5 µL of 0.031% xylose (top row, well 6 from left hand side) and approximately 5 µL of 0.1 U/mL xylanase in the enzyme assay (bottom row, well 7 from left hand side).

### Example ii

The methodology used in this Example is also a detection system based on a reducing sugar methodology. The methodology used in this Example is the same as that for Example i top row above (xylose 1-0.0001%) - except that Tetrazolium Blue was substituted with Nitroblue Tetrazolium and the reaction was allowed to develop at room temperature for 60 minutes. The data are presented in Figure 27.

### Example iii

The methodology described in Example i or Example ii may be used in an assay device or method according to the present invention to detect active xylanase in a sample. In this respect, the xylanase detection methodology is incorporated into a rapid test similar to that described in Part 1 (above).

Thus, in a similar manner to the phytase test mentioned above, the xylanase from a complex feed extract is captured on an antibody raised against a desired or suitable xylanase. In the methodology, the xylanase enzyme is exposed to the xylan substrate and the resulting xylose produced is detected by, for example, the tetrazolium reducing sugar assay. Figure 28 presents a schematic of this methodology.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry or related fields are intended to be within the scope of the following claims.

The invention also relates to the following aspects as defined in the following numbered paragraphs:
1. An assay device (1) for detecting active enzyme in a sample, said device comprising:
   (a) a placement region (10) onto which the sample can be placed;
   (b) a matrix (20) operably connected to said placement region (10) such that the sample when present (such as placed) on said placement region (10) can migrate along said matrix (20);
   (c) at least one distinct capture location (30) on said matrix (20), wherein each distinct capture location (30) is distanced away from the placement region (10), and wherein the sample can migrate across said distinct capture location (30);
   (d) capture means (40) being present at or defining each distinct capture location (30), wherein said capture means (40) are capable of binding to said enzyme such that at least a portion of said sample of said enzyme is retained at at least one distinct capture location (30); and
   (e) selective indication means (50) or at least a component thereof to provide selective indication of the presence of active enzyme bound to said capture means.
2. An assay device (1) according to paragraph 1 wherein said placement region (10) is an absorbent pad.
3. An assay device (1) according to paragraph 1 or paragraph 2 wherein said placement region (10) is at one end of said matrix (20).
4. The assay device (1) of any one of paragraphs 1 to 3 wherein said device comprises multiple distinct capture locations (30).
5. The assay device (1) of any one of paragraphs 1 to 4 wherein said device comprises more than two distinct capture locations (30).
6. The assay device (1) of any one of paragraphs 1 to 5 wherein said device comprises three distinct capture locations (30).
7. The assay device (1) of paragraph 5 or paragraph 6 wherein the amount of capture means (40) at each distinct capture location is different or the same.
8. The assay device (1) of paragraph 7 wherein the amount of capture means at (40) each distinct capture location (30) increases or decreases the further the location is away from the placement region (10).
9. The assay device of any one of paragraphs 1 to 8 wherein said device) comprises a blocking reagent.
10. The assay device (1) of paragraph 9 wherein said blocking reagent is bound directly to said device.
11. The assay device (1) of paragraph 9 or paragraph 10 wherein said blocking reagent is or comprises surfactant.
12. The assay device (1) of any of paragraphs 9 to 11 wherein said blocking reagent is a surfactant.
13. The assay device (1) of paragraph 11 or paragraph 12 wherein said blocking reagent is Tween.
14. The assay device (1) of any of paragraphs 1 to 13 wherein the matrix (20) is an absorbent material.
15. The assay device (1) of any of paragraphs 1 to 14 wherein the matrix (20) is or comprises nitrocellulose.
16. The assay device (1) of any of paragraphs 1 to 15 wherein said selective indication means (50) comprises a precipitation reaction.
17. The assay device (1) of any of paragraphs 1 to 16 wherein said selective indication means (50) comprises or provides a visible colour change.
18. The assay device (1) of paragraph 17 wherein said colour change is caused by reaction of a substrate of the enzyme to form a moiety that reacts with a reactive species.
19. The assay device (1) of paragraph 18 wherein said substrate is bound to said matrix (20).
20. The assay device (1) of paragraph 18 or paragraph 19 wherein said substrate is phosphatase substrate using/having a phosphate donor.
21. The assay device (1) of paragraph 20 wherein said phosphatase substrate is 2-phospho-L-ascorbic acid trisodium (AsAP).
22. The assay device (1) of any one of paragraphs 18 to 21 wherein said reactive species is a tetrazolium compound.
23. The assay device (1) of paragraph 22 wherein said reactive species is a nitroblue tetrazolium compound.
24. The assay device (1) of paragraph 23 wherein said reactive species is nitroblue tetrazolium chloride (NBT).
25. The assay device (1) of paragraph 22 wherein said reactive species is a halonitrotetrazolium compound.
26. The assay device (1) of paragraph 25 wherein said reactive species is an iodonitrotetrazolium compound.
27. The assay device (1) of paragraph 26 wherein said reactive species is iodonitrotetrazolium chloride (INT).
28. The assay device (1) of any one of paragraphs 18 to 27 wherein said phosphatase substrate is reacted with said reactive species in the presence of a reaction enhancer.
29. The assay device (1) of paragraph 28 wherein said reaction enhancer is bound to a secondary matrix.
30. The assay device (1) of paragraph 28 or paragraph 29 wherein said reaction enhancer is a phenazine compound.
31. The assay device (1) of any one of paragraphs 28 to 30 wherein said reaction enhancer is a methosulphate compound.
32. The assay device (1) of any one of paragraphs 28 to 31 wherein said reaction enhancer is phenazine methosulphate (PMS).
33. The assay device (1) of any one of paragraphs 1 to 32 wherein said capture means (40) are the same.
34. The assay device (1) of any one of paragraphs 1 to 33 wherein the capture means (40) are antibodies.
35. The assay device (1) of paragraph 34 wherein said antibodies are antibodies raised against said enzyme or a component thereof (e.g. a fragment thereof and/or an epitope thereof).
36. The assay device (1) of paragraph 34 or paragraph 35 wherein said antibodies are polyclonal or monoclonal antibodies.
37. The assay device (1) of any one of paragraphs 1 to 36 wherein said sample is an aqueous sample.
38. The assay device (1) of any one of paragraphs 1 to 37 wherein said sample is derived from a feed or a feed additive.
39. The assay device (1) of any one of paragraphs 1 to 38 wherein said sample is an extraction from a feed or a feed additive.
40. The assay device (1) of any one of paragraphs 1 to 39 wherein said sample is an extraction from a feed pellet.
41. The assay device (1) of any one of paragraphs 1 to 40 wherein said enzyme is an acid phosphatase.
42. The assay device (1) of any one of paragraphs 1 to 41 wherein said enzyme is a phytase.
43. The assay device (1) of paragraph 42 wherein said phytase is a 6-phytase, such as phytase BP17.
44. The assay device (1) of any one of paragraphs 1 to 41 wherein said enzyme is a xylanase.
45. The assay device (1) of any one of paragraphs 1 to 44 wherein said migration is in a longitudinal direction.
46. The assay device (1) of any one of paragraphs 1 to 45 wherein said capture locations (30) are perpendicular to the migrational flow of the sample.
47. The assay device of any one of paragraphs 1 to 46 wherein said device is a strip or a stick.
48. The assay device of any one of paragraphs 1 to 47 wherein said device comprises a cover.
49. The assay device of paragraph 48 wherein said cover is formed by a fold in the device that defines two sections.
50. The assay device of paragraph 49 wherein some or all of the device components (a) to (e) are present in one section and wherein some or all of the device components (a) to (e) are present in the other section.
51. The assay device of paragraph 49 or paragraph 50 wherein the selective indication means (e), or at least a component thereof is present in one section and wherein some or all of the device components (a) to (d) are present in the same or other section.
52. The assay device of any of paragraphs 49 to 51 wherein the selective indication means (e), or at least a component thereof, is present in one section and wherein the device components (a) to (d) are present in the same or other section.
53. The assay device of any of paragraphs 48 to 52 wherein the selective indication means (e), or at least a component thereof, is present in or on the cover.
54. An assay device (1) for at least semi-quantitatively assessing the level of active phytase in a feed or a feed additive.
55. An assay device (1) for at least semi-quantitatively assessing the level of active xylanase in a feed or a feed additive.
56. A method of determining the presence of active enzyme in a sample wherein the sample is placed on the placement region (10) of the assay device (1) of any one of paragraphs 1 to 55 and the selective indication means (50) of said device indicate if active enzyme is present.
57. A method of determining active enzyme levels in a sample wherein the sample is placed on the placement region (10) of the assay device (1) of any of paragraphs 1 to 55 and the selective indication means (50) of said device indicate the amount of active enzyme present semi-quantitatively.
58. A method of paragraph 56 or 57 wherein the sample is obtained from a food or feed.
59. A method of any of paragraphs 56 to 58 wherein the active enzyme to be detected is phytase.
60. A method of any of paragraphs 56 to 58 wherein the active enzyme to be detected is xylanase.
61. The method of any of paragraphs 56 to 60 wherein the method is used to determine if additional enzyme needs to be added to a food or feed.
62. The method of any of paragraphs 56 to 61 wherein the method is used to determine if additional phytase needs to be added to a food or feed.
63. The method of any of paragraphs 56 to 62 wherein said method is used as a form of quality control.
64. A kit comprising or capable of forming the assay device (1) of any of paragraphs 1 to 55.
65. A kit of paragraph 64 comprising dried reagents which are reconstituted by the addition of a liquid or aqueous sample.
66. A kit of paragraph 64 or 65 further comprising at least one liquid reagent to be added by an operator.
67. A kit of paragraph 66 wherein the liquid reagent is a tetrazolium solution.
68. A method of determining the presence of active enzyme in a sample wherein the method comprises the steps of:
   a) contacting at least a portion of the sample with a capture means whereby the enzyme is captured by said capture means;
   b) providing a suitable substrate for the captured enzyme wherein said substrate reacts with said captured enzyme to yield an enzyme product;
   c) providing a reactive species capable of reacting with the enzyme product wherein said reactive species reacts with said enzyme product to yield an insoluble product; and
   d) detecting the insoluble product obtained in step c) wherein said detecting correlates to the presence and/or quantity of an active enzyme in said sample.
69. A method according to paragraph 68 wherein said enzyme is an enzyme as defined in any one of paragraphs 1 to 67.
70. A method according to paragraph 68 or paragraph 69 wherein said sample is a sample as defined in any one of paragraphs 1 to 67.
71. A method according to any one of paragraphs 68 to 70 wherein said capture means is a capture means as defined in any one of paragraphs 1 to 67.
72. A method according to any one of paragraphs 68 to 71 wherein said reactive species is a reactive species as defined in any one of paragraphs 1 to 67.
73. A method according to any one of paragraphs 68 to 72 wherein said method uses a device as defined in any one of paragraphs 1 to 67.
74. An assay device (1) or a method using same or the use thereof, wherein said assay device is for detecting active phytase in a sample, said device comprising:
   (a) a placement region (10) onto which the sample can be placed;
   (b) a matrix (20) operably connected to said placement region (10) such that the sample when present (such as placed) on said placement region (10) can migrate along said matrix (20);
   (c) at least one distinct capture location (30) on said matrix (20), wherein each distinct capture location (30) is distanced away from the placement region (10), and wherein the sample can migrate across said distinct capture location (30);
   (d) capture means (40) being present at or defining each distinct capture location (30), wherein said capture means (40) are capable of binding to said phytase such that at least a portion of said sample of said phytase is retained at at least one distinct capture location (30); and
   (e) selective indication means (50), or at least a component thereof, to provide selective indication of the presence of active phytase bound to said capture means.
75. An assay device (1) or a method using same or the use thereof, wherein said assay device is for detecting active phytase in a sample, said device comprising:
   (a) a placement region (10) onto which the sample can be placed;
   (b) a matrix (20) operably connected to said placement region (10) such that the sample when present (such as placed) on said placement region (10) can migrate along said matrix (20);
   (c) at least one distinct capture location (30) on said matrix (20), wherein each distinct capture location (30) is distanced away from the placement region (10), and wherein the sample can migrate across said distinct capture location (30);
   (d) capture means (40) being present at or defining each distinct capture location (30), wherein said capture means (40) are capable of binding to said phytase such that at least a portion of said sample of said phytase is retained at at least one distinct capture location (30), wherein said capture means comprises an antibody; and
   (e) selective indication means (50), or at least a component thereof, to provide selective indication of the presence of active phytase bound to said capture means.
76. An assay device (1) or a method using same or the use thereof, wherein said assay device is for detecting active phytase in a sample, said device comprising:
   (a) a placement region (10) onto which the sample can be placed;
   (b) a matrix (20) operably connected to said placement region (10) such that the sample when present (such as placed) on said placement region (10) can migrate along said matrix (20);
   (c) at least one distinct capture location (30) on said matrix (20), wherein each distinct capture location (30) is distanced away from the placement region (10), and wherein the sample can migrate across said distinct capture location (30);
   (d) capture means (40) being present at or defining each distinct capture location (30), wherein said capture means (40) are capable of binding to said phytase such that at least a portion of said sample of said phytase is retained at at least one distinct capture location (30), wherein said capture means comprises an antibody that can bind BP17; and
   (e) selective indication means (50), or at least a component thereof, to provide selective indication of the presence of active phytase bound to said capture means.
77. An assay device or a method using same or the use thereof, wherein said assay device is for detecting active xylanase in a sample, said device comprising:
   (a) a placement region onto which the sample can be placed;
   (b) a matrix operably connected to said placement region such that the sample when present (such as placed) on said placement region can migrate along said matrix;
   (c) at least one distinct capture location on said matrix, wherein each distinct capture location is distanced away from the placement region, and wherein the sample can migrate across said distinct capture location;
   (d) capture means being present at or defining each distinct capture location, wherein said capture means are capable of binding to said xylanase such that at least a portion of said sample of said xylanase is retained at at least one distinct capture location; and
   (e) selective indication means, or at least a component thereof, to provide selective indication of the presence of active xylanase bound to said capture means.
78. An assay device or a method using same or the use thereof, wherein said assay device is for detecting active xylanase in a sample, said device comprising:
   (a) a placement region onto which the sample can be placed;
   (b) a matrix operably connected to said placement region such that the sample when present (such as placed) on said placement region can migrate along said matrix;
   (c) at least one distinct capture location on said matrix, wherein each distinct capture location is distanced away from the placement region, and wherein the sample can migrate across said distinct capture location;
   (d) capture means being present at or defining each distinct capture location, wherein said capture means are capable of binding to said xylanase such that at least a portion of said sample of said xylanase is retained at at least one distinct capture location, wherein said capture means comprises an antibody; and
   (e) selective indication means, or at least a component thereof, to provide selective indication of the presence of active xylanase bound to said capture means.
79. An assay device or a method using same or the use thereof according to any one of the preceding paragraphs wherein said selective indication means are constituted on and/or in the assay device when the assay device is in use.
80. A kit comprising or capable of forming the assay device (1) of any one of the preceding paragraphs wherein the selective indication means (50) is added to the kit.
81. A kit comprising or capable of forming the assay device (1) of any one of the preceding paragraphs wherein at least one component of the selective indication means (50) is added to the kit.
82. A kit of any one of the preceding paragraphs supplied with a score card or reference card.
83. An assay device (1) substantially as described herein and with reference to the drawings.
84. A method substantially as described herein and with reference to the drawings.
85. A use substantially as described herein and with reference to the drawings.
86. A kit substantially as described herein and with reference to the drawings.

## Claims

1. An assay device (1) for detecting active enzyme in a sample, said device comprising:
(a) a placement region (10) onto which the sample can be placed, preferably wherein said placement region (10) is at one end of said matrix (20);
(b) a matrix (20) operably connected to said placement region (10) such that the sample when present (such as placed) on said placement region (10) can migrate along said matrix (20);
(c) at least one distinct capture location (30) on said matrix (20), wherein each distinct capture location (30) is distanced away from the placement region (10), and wherein the sample can migrate across said distinct capture location (30);
(d) capture means (40) being present at or defining each distinct capture location (30), wherein said capture means (40) are capable of binding to said enzyme such that at least a portion of said sample of said enzyme is retained at at least one distinct capture location (30); and
(e) selective indication means (50) or at least a component thereof to provide selective indication of the presence of active enzyme bound to said capture means.

2. The assay device of claim 1 wherein said device (1) comprises a blocking reagent, preferably wherein said blocking reagent is bound directly to said device.

3. The assay device (1) of claim 1 or claim 2 wherein said selective indication means (50) comprisesC
a precipitation reaction; or
provides a visible colour change.

4. The assay device (1) of claim 3 wherein said colour change is caused by reaction of a substrate of the enzyme to form a moiety that reacts with a reactive species, preferably wherein said substrate is bound to said matrix (20),
preferably wherein said substrate is phosphatase substrate using/having a phosphate donor,
preferably wherein said phosphatase substrate is 2-phospho-L-ascorbic acid trisodium (AsAP).

5. The assay device (1) of claim 4 wherein said reactive species is a tetrazolium compound.

6. The assay device (1) of any one of claims 1 to 5 wherein the capture means (40) are antibodies,
preferably wherein said antibodies are antibodies raised against said enzyme or a component thereof (e.g. a fragment thereof and/or an epitope thereof),
preferably wherein said antibodies are polyclonal or monoclonal antibodies.

7. The assay device (1) of any one of claims 1 to 6 wherein said enzyme is
an acid phosphatase; and/or
a phytase, preferably a 6-phytase, such as phytase BP17; and/or
a xylanase.

8. An assay device (1) for at least semi-quantitatively assessing the level of active phytase or active xylanase in a feed or a feed additive.

9. A method of determining the presence of active enzyme in a sample wherein the sample is placed on the placement region (10) of the assay device (1) of any one of claims 1 to 7 and the selective indication means (50) of said device indicate if active enzyme is present, preferably wherein the active enzyme to be detected is phytase or xylanase.

10. A method of determining active enzyme levels in a sample wherein the sample is placed on the placement region (10) of the assay device (1) of any of claims 1 to 7 and the selective indication means (50) of said device indicate the amount of active enzyme present semi-quantitatively, preferably wherein the active enzyme to be detected is phytase or xylanase.

11. A kit comprising or capable of forming the assay device (1) of any of claims 1 to 7.

12. A method of determining the presence of active enzyme in a sample wherein the method comprises the steps of:
a) contacting at least a portion of the sample with a capture means whereby the enzyme is captured by said capture means;
b) providing a suitable substrate for the captured enzyme wherein said substrate reacts with said captured enzyme to yield an enzyme product;
c) providing a reactive species capable of reacting with the enzyme product wherein said reactive species reacts with said enzyme product to yield an insoluble product; and
d) detecting the insoluble product obtained in step c) wherein said detecting correlates to the presence and/or quantity of an active enzyme in said sample,
preferably wherein
said enzyme is an enzyme as defined in any one of claims 1 to 11; and/or
said sample is a sample as defined in any one of claims 1 to 11; and/or
said capture means is a capture means as defined in any one of claims 1 to 11; and/or
said reactive species is a reactive species as defined in any one of claims 1 to 11; and/or
said method uses a device as defined in any one of claims 1 to 11.

13. An assay device (1) or a method using same or the use thereof, wherein said assay device is for detecting active phytase in a sample, said device comprising:
(a) a placement region (10) onto which the sample can be placed;
(b) a matrix (20) operably connected to said placement region (10) such that the sample when present (such as placed) on said placement region (10) can migrate along said matrix (20);
(c) at least one distinct capture location (30) on said matrix (20), wherein each distinct capture location (30) is distanced away from the placement region (10), and wherein the sample can migrate across said distinct capture location (30);
(d) capture means (40) being present at or defining each distinct capture location (30), wherein said capture means (40) are capable of binding to said phytase such that at least a portion of said sample of said phytase is retained at at least one distinct capture location (30), preferably wherein said capture means comprises an antibody, preferably wherein said antibody can bind BP17; and
(e) selective indication means (50), or at least a component thereof, to provide selective indication of the presence of active phytase bound to said capture means.

14. An assay device or a method using same or the use thereof, wherein said assay device is for detecting active xylanase in a sample, said device comprising:
(a) a placement region onto which the sample can be placed;
(b) a matrix operably connected to said placement region such that the sample when present (such as placed) on said placement region can migrate along said matrix;
(c) at least one distinct capture location on said matrix, wherein each distinct capture location is distanced away from the placement region, and wherein the sample can migrate across said distinct capture location;
(d) capture means being present at or defining each distinct capture location, wherein said capture means are capable of binding to said xylanase such that at least a portion of said sample of said xylanase is retained at at least one distinct capture location, preferably wherein said capture means comprises an antibody; and
(e) selective indication means, or at least a component thereof, to provide selective indication of the presence of active xylanase bound to said capture means.

15. A kit comprising or capable of forming the assay device (1) of any one of the preceding claims wherein
the selective indication means (50) is added to the kit; or
at least one component of the selective indication means (50) is added to the kit.
